(19) Europäisches Patentamt
European Patent Office
Office européen des brevets

(11) EP 4 696 332 A1

(12) EUROPEAN PATENT APPLICATION
published in accordance with Art. 153(4) EPC

(43) Date of publication:
**18.02.2026 Bulletin 2026/08**

(21) Application number: **24788709.4**

(22) Date of filing: **09.04.2024**

(51) International Patent Classification (IPC):
***A61K 47/68*** *(2017.01)* ***A61K 31/517*** *(2006.01)*

(52) Cooperative Patent Classification (CPC):
**A61K 31/4745; A61K 31/517; A61K 31/5377; A61K 31/5383; A61K 39/395; A61K 45/00; A61K 47/65; A61K 47/68; A61P 1/00; A61P 1/16; A61P 1/18; A61P 11/00; A61P 13/00; A61P 13/08; A61P 13/10;** (Cont.)

(86) International application number:
**PCT/JP2024/014351**

(87) International publication number:
**WO 2024/214685 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.04.2023 JP 2023063372**

(71) Applicant: **Daiichi Sankyo Company, Limited**
**Tokyo 103-8426 (JP)**

(72) Inventors:
- **KATSUMATA Len**
  **Tokyo 103-8426 (JP)**
- **HASEGAWA Jun**
  **Tokyo 103-8426 (JP)**
- **HATTORI Chiharu**
  **Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

# (54) COMBINATION OF ANTI-B7-H3 ANTIBODY-DRUG CONJUGATE WITH ATR INHIBITOR OR ATM INHIBITOR

(57) In some aspects, an object of the present invention is to provide a pharmaceutical composition and a method of treatment comprising administering a specific anti-B7-H3 antibody-drug conjugate in combination with an ATR inhibitor or an ATM inhibitor.

[Solution]

In some aspects, provided is a pharmaceutical composition and a method of treatment, comprising administering an antibody-drug conjugate, in which a drug-linker represented by the following formula:

EP 4 696 332 A1

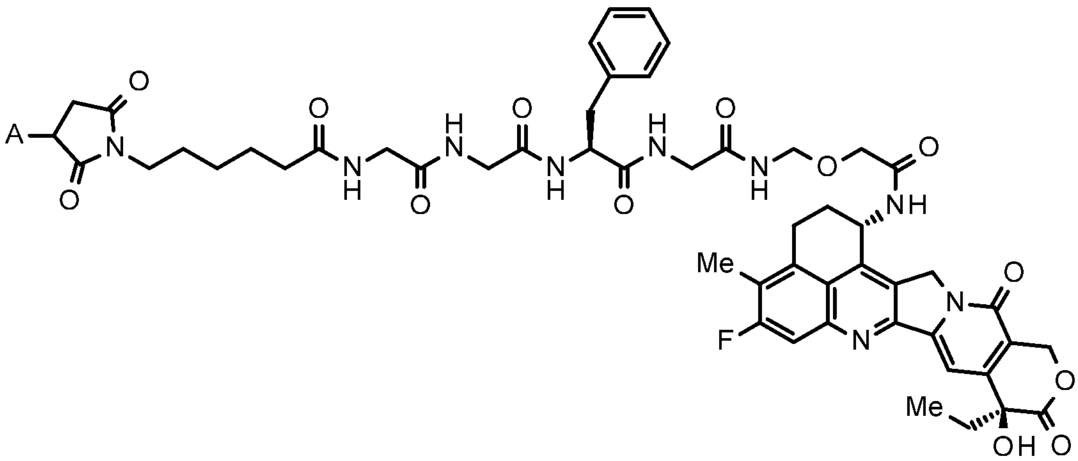

wherein A represents a connecting position to an anti-B7-H3 antibody,
and the anti-B7-H3 antibody are conjugated via a thioether bond, in combination with an ATR inhibitor or an ATM inhibitor.

Fig. 16

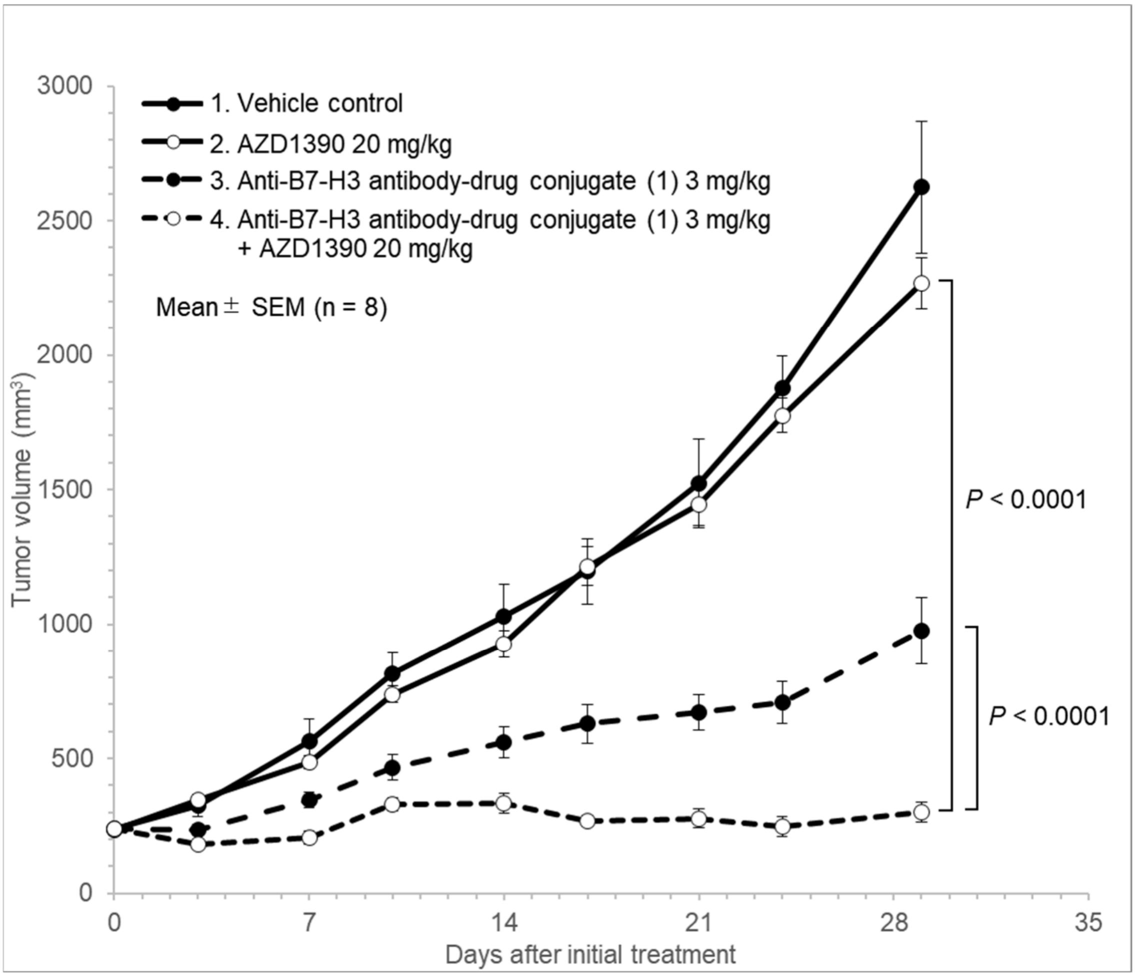


(52) Cooperative Patent Classification (CPC): (Cont.)
**A61P 13/12; A61P 15/00; A61P 17/00; A61P 19/00; A61P 21/00; A61P 35/00; A61P 35/02; A61P 43/00; C07K 16/00; C07K 16/28**

## Description

[Technical Field]

In some aspects, the present invention relates to a pharmaceutical composition comprising administering a specific anti-B7-H3 antibody-drug conjugate in combination with an ATR inhibitor or an ATM inhibitor, and/or a method of treatment comprising administering a specific anti-B7-H3 antibody-drug conjugate in combination with an ATR inhibitor or an ATM inhibitor to a subject.

[Background Art]

Various pathways of DNA damage response (DDR) are known, of which the pathway via Ataxia telangiectasia-mutated (ATM) and the pathway via Ataxia-telangiectasia mutated and Rad3-related (ATR) are important in responding to the signaling system of DNA strand breakage. Inhibitors of the major molecules of these pathways (ATM inhibitor, ATR inhibitor) inhibit the DNA damage reponse and thereby depress cell functions, whereby a therapeutic effect on cancer is expected (Non Patent Reference 1).

An antibody-drug conjugate (ADC), having a cytotoxic drug conjugated to an antibody capable of binding to an antigen expressed on the surface of cancer cells and cellular internalization, can deliver the drug selectively to cancer cells and can thus cause accumulation of the drug within cancer cells and kill the cancer cells. As one such antibody-drug conjugate, an antibody-drug conjugate comprising an anti-B7-H3 antibody and a derivative of exatecan, which is a topoisomerase I inhibitor, as its components is known (Patent References 1).

However, the effect gained when the above anti-B7-H3 antibody-drug conjugate is used in combination with an ATR inhibitor or an ATM inhibitor is not known.

[Citation List]

[Patent Literature]

[Patent Reference 1] International Publication No. WO 2014/057687

[Non Patent Literature]

[Non Patent Reference 1] Cancer Treat. Rev. 60, 139-151 (2017).

[Summary of Invention]

[Technical Problem]

In some aspects, the anti-B7-H3 antibody-drug conjugate used in the present invention (the anti-B7-H3 antibody-drug conjugate comprising a derivative of exatecan as its component) has been confirmed to exhibit an excellent antitumor effect even when used as a single agent. However, establishment of a method of treatment capable of suppressing cancer cell growth combinedly and thereby exhibiting a more superior antitumor effect is desired by using such a conjugate in combination with other anticancer agent(s) having a different mechanism of action.

In some aspects, an object of the present invention is to provide a pharmaceutical composition comprising administering a specific anti-B7-H3 antibody-drug conjugate in combination with an ATR inhibitor or an ATM inhibitor, and/or a method of treatment comprising administering a specific anti-B7-H3 antibody-drug conjugate in combination with an ATR inhibitor or an ATM inhibitor to a subject.

[Solution to Problem]

The present inventors conducted extensive studies to achieve the above object and found that when a specific anti-B7-H3 antibody-drug conjugate is administered in combination with an ATR inhibitor or an ATM inhibitor, an excellent combination use effect is demonstrated, whereby the present invention has been accomplished.

Thus, in some embodiments, the present invention provides the followings:

[1] A pharmaceutical composition comprising an anti-B7-H3 antibody-drug conjugate, wherein the anti-B7-H3 antibody-drug conjugate is administered in combination with an ATR inhibitor or an ATM inhibitor; and the anti-B7-H3 antibody-drug conjugate is an antibody-drug conjugate in which a drug-linker represented by the following

formula:

[Formula 1]

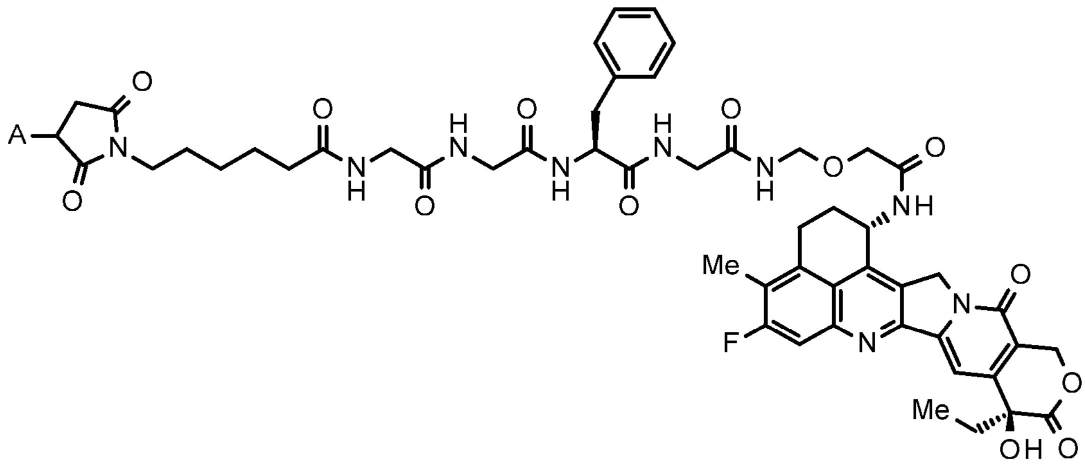

wherein A represents a connecting position to an anti-B7-H3 antibody or functional fragment thereof, and the anti-B7-H3 antibody or functional fragment thereof are conjugated via a thioether bond.

[2] A pharmaceutical composition comprising an anti-B7-H3 antibody-drug conjugate, wherein the anti-B7-H3 antibody-drug conjugate is administered in combination with an ATR inhibitor or an ATM inhibitor; and the anti-B7-H3 antibody-drug conjugate comprises an anti-B7-H3 antibody or functional fragment thereof conjugated to the drug-linker represented by the above Formula 1 via the thioether bond.
[3] The pharmaceutical composition according to [1] or [2], for treating cancer.
[4] The pharmaceutical composition according to any one of [1] to [3], wherein the anti-B7-H3 antibody or functional fragment thereof comprises CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, CDRH3 consisting of an amino acid sequence consisting of amino acid residues 118 to 130 of SEQ ID NO: 3, CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.
[5] The pharmaceutical composition according to any one of [1] to [4], wherein the anti-B7-H3 antibody or functional fragment thereof comprises a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 8, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 9, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 10, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 4, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 11, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 12, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 13, a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 8, a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 9, a heavy chain variable region consisting of an amino acid sequence consisting of amino acid residues 20 to 141 of SEQ ID NO: 7 and a light chain variable region consisting of an amino

acid sequence consisting of amino acid residues 21 to 128 of SEQ ID NO: 10, or a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 4.

[6] The pharmaceutical composition according to [5], wherein the anti-B7-H3 antibody or functional fragment thereof comprises a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 4.

[7] The pharmaceutical composition according to any one of [1] to [5], wherein the anti-B7-H3 antibody comprises (or consists of) a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

[8] The pharmaceutical composition according to [7], wherein the anti-B7-H3 antibody comprises (or consists of) a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[9] The pharmaceutical composition according to any one of [1] to [8], wherein a lysine residue at the carboxyl terminus of the heavy chain of the anti-B7-H3 antibody is deleted.

[10] The pharmaceutical composition according to any one of [1] to [9], wherein the average number of units of the drug-linker conjugated per antibody molecule in the anti-B7-H3 antibody-drug conjugate is in the range of from 3.5 to 4.5.

[11] The pharmaceutical composition according to any one of [1] to [9], wherein the number of units of the drug-linker conjugated per antibody molecule in the anti-B7-H3 antibody-drug conjugate is an integer in the range from preferably 2 to 8, more preferably selected from 2, 4, 6, or 8, and most preferably 4.

[12] The pharmaceutical composition according to any one of [1] to [11], wherein the anti-B7-H3 antibody-drug conjugate is ifinatamab deruxtecan.

[13] The pharmaceutical composition according to any one of [1] to [12], wherein the ATR inhibitor is AZD6738, BAY-1895344 or ETP-46464, or a pharmaceutically acceptable salt thereof.

[14] The pharmaceutical composition according to any one of [1] to [13], wherein the ATR inhibitor is AZD6738 or a pharmaceutically acceptable salt thereof.

[15] The pharmaceutical composition according to any one of [1] to [12], wherein the ATM inhibitor is AZD1390, AZD0156, KU-55933, KU-60019, KU-59403, CP466722 or NVP-BEZ235, or a pharmaceutically acceptable salt thereof.

[16] The pharmaceutical composition according to any one of [1] to [12] or [15], wherein the ATM inhibitor is AZD1390 or a pharmaceutically acceptable salt thereof.

[17] The pharmaceutical composition according to any one of [1] to [16], wherein the anti-B7-H3 antibody-drug conjugate and the ATR inhibitor or ATM inhibitor are contained in separate formulations and are administered at the same time or different times.

[18] The pharmaceutical composition according to any one of [1] to [17], for treating cancer selected from the group consisting of breast cancer, stomach cancer, colorectal cancer, lung cancer, esophageal cancer, head and neck cancer, gastroesophageal junction adenocarcinoma, biliary tract cancer, Paget’s disease, pancreatic cancer, ovarian

cancer, uterine carcinosarcoma, urothelial cancer, prostate cancer, bladder cancer, gastrointestinal stromal tumor, cervical cancer, squamous cell cancer, peritoneal cancer, liver cancer, hepatocellular cancer, uterine cancer, kidney cancer, vulvar cancer, thyroid cancer, penile cancer, leukemia, malignant lymphoma, plasmacytoma, myeloma, glioblastoma multiforme, osteosarcoma, sarcoma, melanoma, and mesothelioma.

[19] The pharmaceutical composition according to any one of [1] to [18], for treating cancer selected from the group consisting of breast cancer, lung cancer, esophageal cancer, head and neck cancer, prostate cancer, and squamous cell cancer.

[20] A pharmaceutical composition comprising an anti-B7-H3 antibody-drug conjugate, wherein the anti-B7-H3 antibody-drug conjugate is administered in combination with an ATR inhibitor or an ATM inhibitor, and the anti-B7-H3 antibody-drug conjugate is an antibody-drug conjugate represented by the following formula:

[Formula 2]

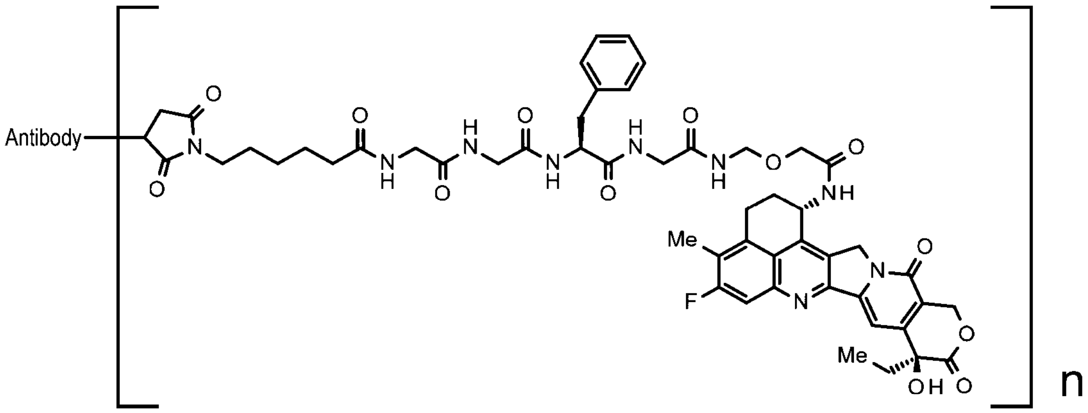

wherein 'Antibody' is an anti-B7-H3 antibody or functional fragment thereof; a drug-linker is conjugated to the antibody or functional fragment thereof via a thioether bond; and n represents the average number of units of the drug-linker conjugated per antibody molecule.

[21] A pharmaceutical composition comprising an anti-B7-H3 antibody-drug conjugate, wherein the anti-B7-H3 antibody-drug conjugate is administered in combination with an ATR inhibitor or an ATM inhibitor, and the anti-B7-H3 antibody-drug conjugate is represented by the above Formula 2, wherein 'Antibody' is an anti-B7-H3 antibody or functional fragment thereof; each drug-linker represented by the structure shown within the bracket in the above Formula 2 is conjugated to the antibody or functional fragment thereof via a thioether bond; and n represents the average number of units of the drug-linker conjugated per antibody molecule.

[22] The pharmaceutical composition according to [20] or [21], for treating cancer.

[23] The pharmaceutical composition according to any one of [20] to [22], wherein the anti-B7-H3 antibody comprises a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[24] The pharmaceutical composition according to any one of [20] to [23], wherein a lysine residue at the carboxyl terminus of the heavy chain of the anti-B7-H3 antibody is deleted.

[25] The pharmaceutical composition according to any one of [20] to [24], wherein n is in the range of from 3.5 to 4.5.

[26] The pharmaceutical composition according to any one of [20] to [25], wherein the anti-B7-H3 antibody-drug conjugate is ifinatamab deruxtecan.

[27] The pharmaceutical composition according to any one of [20] to [26], wherein the ATR inhibitor is AZD6738, BAY-1895344 or ETP-46464, or a pharmaceutically acceptable salt thereof.

[28] The pharmaceutical composition according to any one of [20] to [27], wherein the ATR inhibitor is AZD6738 or a pharmaceutically acceptable salt thereof.

[29] The pharmaceutical composition according to any one of [20] to [26], wherein the ATM inhibitor is AZD1390, AZD0156, KU-55933, KU-60019, KU-59403, CP466722 or NVP-BEZ235, or a pharmaceutically acceptable salt thereof.

[30] The pharmaceutical composition according to any one of [20] to [26] or [29], wherein the ATM inhibitor is AZD1390 or a pharmaceutically acceptable salt thereof.

[31] The pharmaceutical composition according to any one of [20] to [30], wherein the anti-B7-H3 antibody-drug conjugate and the ATR inhibitor or ATM inhibitor are contained in separate formulations and are administered at the same time or different times.

[32] The pharmaceutical composition according to any one of [20] to [31], for treating cancer selected from the group consisting of breast cancer, stomach cancer, colorectal cancer, lung cancer, esophageal cancer, head and neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, cervical cancer, squamous cell cancer, peritoneal cancer, liver cancer, hepatocellular cancer, uterine cancer, kidney cancer, vulvar cancer, thyroid cancer, penile cancer, leukemia, malignant lymphoma, plasmacytoma, myeloma, glioblastoma multiforme, osteosarcoma, sarcoma, melanoma, and mesothelioma.

[33] The pharmaceutical composition according to any one of [20] to [32], for treating cancer selected from the group consisting of breast cancer, lung cancer, esophageal cancer, head and neck cancer, prostate cancer, and squamous cell cancer.

[34] A method of treatment comprising administering an anti-B7-H3 antibody-drug conjugate in combination with an ATR inhibitor or an ATM inhibitor to a subject in need of treatment, wherein the antibody-drug conjugate is an antibody-drug conjugate in which a drug-linker represented by the following formula:

[Formula 3]

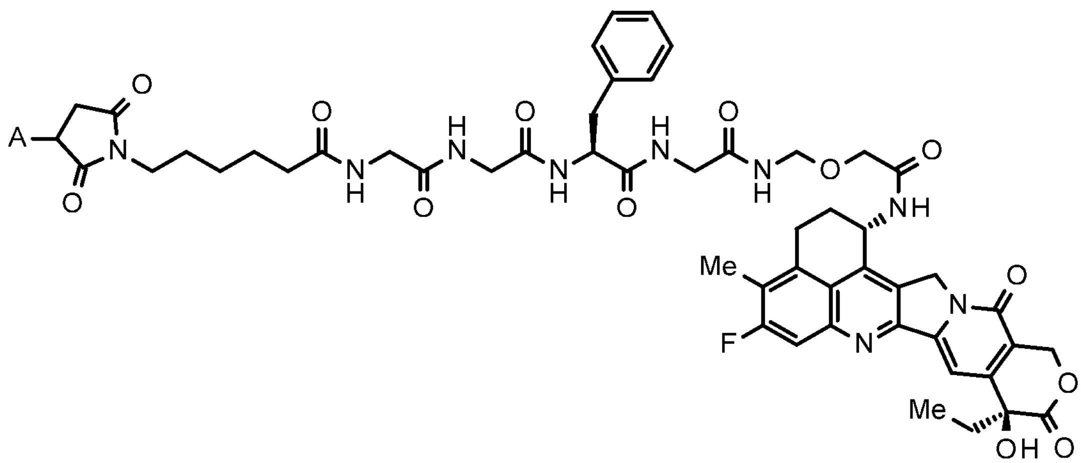

wherein A represents a connecting position to an anti-B7-H3 antibody or functional fragment thereof, and the anti-B7-H3 antibody or functional fragment thereof are conjugated via a thioether bond.

[35] The method of treatment according to [34], wherein the treatment is cancer treatment.

[36] The method of treatment according to [34] or [35], wherein the anti-B7-H3 antibody or functional fragment thereof comprises CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, CDRH3 consisting of an amino acid sequence consisting of amino acid residues 118 to 130 of SEQ ID NO: 3, CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

[37] The method of treatment according to any one of [34] to [36], wherein the anti-B7-H3 antibody or functional fragment thereof comprises a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 8, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 9, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 10, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 4, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 11, a heavy chain variable region consisting of an amino acid sequence consisting of amino acid residues 20 to 141 of SEQ ID NO: 3 and a light chain variable region consisting of an amino

acid sequence consisting of amino acid residues 21 to 128 of SEQ ID NO: 12, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 13, a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 8, a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 9, a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 10, or a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 4.

[38] The method of treatment according to [37], wherein the anti-B7-H3 antibody or functional fragment thereof comprises a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 4.

[39] The method of treatment according to any one of [34] to [37], wherein the anti-B7-H3 antibody comprises (or consists of) a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

[40] The method of treatment according to [39], wherein the anti-B7-H3 antibody comprises (or consists of) a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[41] The method of treatment according to any one of [34] to [40], wherein a lysine residue at the carboxyl terminus of the heavy chain of the anti-B7-H3 antibody is deleted.

[42] The method of treatment according to any one of [34] to [41], wherein the average number of units of the drug-linker conjugated per antibody molecule in the anti-B7-H3 antibody-drug conjugate is in the range of from 3.5 to 4.5.

[43] The method of treatment according to any one of [34] to [42], wherein the anti-B7-H3 antibody-drug conjugate is ifinatamab deruxtecan.

[44] The method of treatment according to any one of [34] to [43], wherein the ATR inhibitor is AZD6738, BAY-1895344 or ETP-46464, or a pharmaceutically acceptable salt thereof.

[45] The method of treatment according to any one of [34] to [44], wherein the ATR inhibitor is AZD6738 or a pharmaceutically acceptable salt thereof.

[46] The method of treatment according to any one of [34] to [43], wherein the ATM inhibitor is AZD1390, AZD0156, KU-55933, KU-60019, KU-59403, CP466722 or NVP-BEZ235, or a pharmaceutically acceptable salt thereof.

[47] The method of treatment according to any one of [34] to [43] or [46], wherein the ATM inhibitor is AZD1390 or a pharmaceutically acceptable salt thereof.

[48] The method of treatment according to any one of [34] to [47], wherein the anti-B7-H3 antibody-drug conjugate and the ATR inhibitor or ATM inhibitor are contained in separate formulations and are administered at the same time or

different times.
[49] The method of treatment according to any one of [34] to [48], for treating cancer selected from the group consisting of breast cancer, stomach cancer, colorectal cancer, lung cancer, esophageal cancer, head and neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, cervical cancer, squamous cell cancer, peritoneal cancer, liver cancer, hepatocellular cancer, uterine cancer, kidney cancer, vulvar cancer, thyroid cancer, penile cancer, leukemia, malignant lymphoma, plasmacytoma, myeloma, glioblastoma multiforme, osteosarcoma, sarcoma, melanoma, and mesothelioma.
[50] The method of treatment according to any one of [34] to [49], for treating cancer selected from the group consisting of breast cancer, lung cancer, esophageal cancer, head and neck cancer, prostate cancer, and squamous cell cancer.
[51] A method of treatment comprising administering an anti-B7-H3 antibody-drug conjugate in combination with an ATR inhibitor or an ATM inhibitor to a subject in need of treatment, wherein the anti-B7-H3 antibody-drug conjugate is an antibody-drug conjugate represented by the following formula:

[Formula 4]

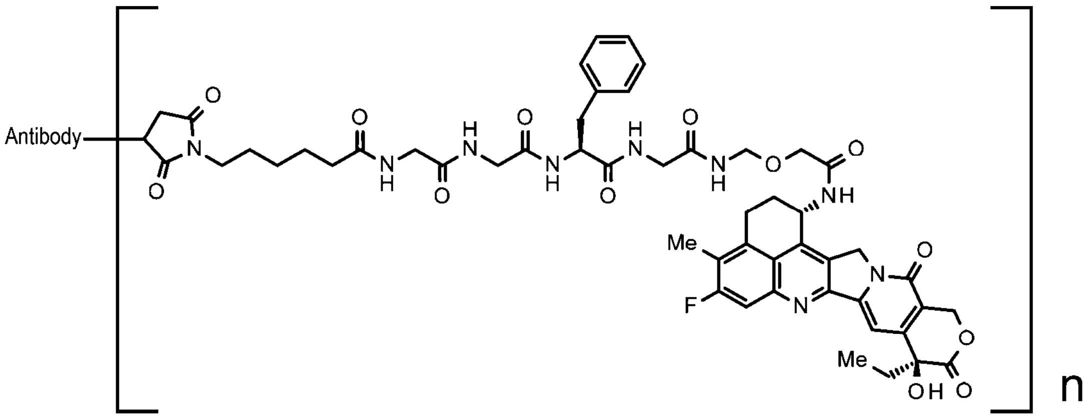

wherein 'Antibody' is an anti-B7-H3 antibody or functional fragment thereof; a drug-linker is conjugated to the antibody or functional fragment thereof via a thioether bond; and n represents the average number of units of the drug-linker conjugated per antibody molecule.
[52] The method of treatment according to [51], wherein the treatment is cancer treatment.
[53] The method of treatment according to [51] or [52], wherein the anti-B7-H3 antibody comprises a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.
[54] The method of treatment according to any one of [51] to [53], wherein a lysine residue at the carboxyl terminus of the heavy chain of the anti-B7-H3 antibody is deleted.
[55] The method of treatment according to any one of [51] to [54], wherein n is in the range of from 3.5 to 4.5.
[56] The method of treatment according to any one of [51] to [55], wherein the anti-B7-H3 antibody-drug conjugate is ifinatamab deruxtecan.
[57] The method of treatment according to any one of [51] to [56], wherein the ATR inhibitor is AZD6738, BAY-1895344 or ETP-46464, or a pharmaceutically acceptable salt thereof.
[58] The method of treatment according to any one of [51] to [57], wherein the ATR inhibitor is AZD6738 or a pharmaceutically acceptable salt thereof.
[59] The method of treatment according to any one of [51] to [56], wherein the ATM inhibitor is AZD1390, AZD0156, KU-55933, KU-60019, KU-59403, CP466722 or NVP-BEZ235, or a pharmaceutically acceptable salt thereof.
[60] The method of treatment according to any one of [51] to [56] or [59], wherein the ATM inhibitor is AZD1390 or a pharmaceutically acceptable salt thereof.
[61] The method of treatment according to any one of [51] to [60], wherein the anti-B7-H3 antibody-drug conjugate and the ATR inhibitor or ATM inhibitor are contained in separate formulations and are administered at the same time or different times.
[62] The method of treatment according to any one of [51] to [61], for treating cancer selected from the group consisting of breast cancer, stomach cancer, colorectal cancer, lung cancer, esophageal cancer, head and neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, cervical

cancer, squamous cell cancer, peritoneal cancer, liver cancer, hepatocellular cancer, uterine cancer, kidney cancer, vulvar cancer, thyroid cancer, penile cancer, leukemia, malignant lymphoma, plasmacytoma, myeloma, glioblastoma multiforme, osteosarcoma, sarcoma, melanoma, and mesothelioma.

[63] The method of treatment according to any one of [51] to [62], for treating cancer selected from the group consisting of breast cancer, lung cancer, esophageal cancer, head and neck cancer, prostate cancer, and squamous cell cancer.

[64] Use of an anti-B7-H3 antibody-drug conjugate, preferably the anti-B7-H3 antibody-drug conjugate defined in any one of [1] to [12], in the manufacture of a medicament for treating cancer, for use in combination with an ATR inhibitor, preferably the ATR inhibitor defined in [13] or [14], or an ATM inhibitor, preferably the ATM inhibitor defined in [15] or [16].

[65] A pharmaceutical combination of (i) an anti-B7-H3 antibody-drug conjugate, preferably the anti-B7-H3 antibody-drug conjugate defined in any one of [1] to [12], and (ii) an ATR inhibitor, preferably the ATR inhibitor defined in [13] or [14], or an ATM inhibitor, preferably the ATM inhibitor defined in [15] or [16].

[66] A pharmaceutical composition comprising (i) an anti-B7-H3 antibody-drug conjugate, preferably the anti-B7-H3 antibody-drug conjugate defined in any one of [1] to [12], and (ii) an ATR inhibitor, preferably the ATR inhibitor defined in [13] or [14], or an ATM inhibitor, preferably the ATM inhibitor defined in [15] or [16], for administration in combination.

[67] A pharmaceutical composition comprising an anti-B7-H3 antibody-drug conjugate, wherein the anti-B7-H3 antibody-drug conjugate is administered in combination with an ATR inhibitor or an ATM inhibitor.

[68] The pharmaceutical composition according to [67], wherein the drug in the anti-B7-H3 antibody-drug conjugate is a topoisomerase I inhibitor.

[69] The pharmaceutical composition according to [68], wherein the drug represented by the following formula is released to express an antitumor activity:

[Formula 5]

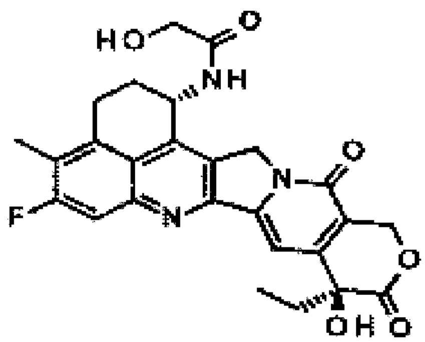

.

[70] The pharmaceutical composition according to [68], wherein an antitumor compound represented by the following formula is conjugated to an anti-B7-H3 antibody through a linker with the nitrogen atom of the amino group at position 1 as the connecting position:

[Formula 6]

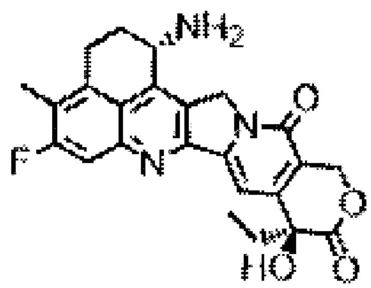

.

[71] The pharmaceutical composition according to [70], wherein the antitumor compound is conjugated to the anti-B7-H3 antibody through the linker via a thioether bond which is formed at a disulfide bond moiety present in the anti-B7-H3 antibody.

[72] The pharmaceutical composition according to [70] or [71], wherein the linker comprises a tetrapeptide residue of

-Gly-Gly-Phe-Gly-.

[Advantageous Effect of Invention]

In some aspects, the present invention provides a pharmaceutical composition comprising administering a specific anti-B7-H3 antibody-drug conjugate in combination with an ATR inhibitor or an ATM inhibitor, and/or a method of treatment comprising administering a specific anti-B7-H3 antibody-drug conjugate in combination with an ATR inhibitor or an ATM inhibitor to a subject.

[Brief Description of Drawings]

[Figure 1] Figure 1 is a diagram showing the amino acid sequence of B7-H3 variant 1 (SEQ ID NO: 1).
[Figure 2] Figure 2 is a diagram showing the amino acid sequence of B7-H3 variant 2 (SEQ ID NO: 2).
[Figure 3] Figure 3 is a diagram showing the amino acid sequence of the heavy chain of the anti-B7-H3 antibody (M30-H1 type) (SEQ ID NO: 3).
[Figure 4] Figure 4 is a diagram showing the amino acid sequence of the light chain of the anti-B7-H3 antibody (M30-L4 type) (SEQ ID NO: 4).
[Figure 5] Figure 5 is a diagram showing the amino acid sequence of the heavy chain of the anti-B7-H3 antibody (M30-H2 type) (SEQ ID NO: 5).
[Figure 6] Figure 6 is a diagram showing the amino acid sequence of the heavy chain of the anti-B7-H3 antibody (M30-H3 type) (SEQ ID NO: 6).
[Figure 7] Figure 7 is a diagram showing the amino acid sequence of the heavy chain of the anti-B7-H3 antibody (M30-H4 type) (SEQ ID NO: 7).
[Figure 8] Figure 8 is a diagram showing the amino acid sequence of the light chain of the anti-B7-H3 antibody (M30-L1 type) (SEQ ID NO: 8).
[Figure 9] Figure 9 is a diagram showing the amino acid sequence of the light chain of the anti-B7-H3 antibody (M30-L2 type) (SEQ ID NO: 9).
[Figure 10] Figure 10 is a diagram showing the amino acid sequence of the light chain of the anti-B7-H3 antibody (M30-L3 type) (SEQ ID NO: 10).
[Figure 11] Figure 11 is a diagram showing the amino acid sequence of the light chain of the anti-B7-H3 antibody (M30-L5 type) (SEQ ID NO: 11).
[Figure 12] Figure 12 is a diagram showing the amino acid sequence of the light chain of the anti-B7-H3 antibody (M30-L6 type) (SEQ ID NO: 12).
[Figure 13] Figure 13 is a diagram showing the amino acid sequence of the light chain of the anti-B7-H3 antibody (M30-L7 type) (SEQ ID NO: 13).

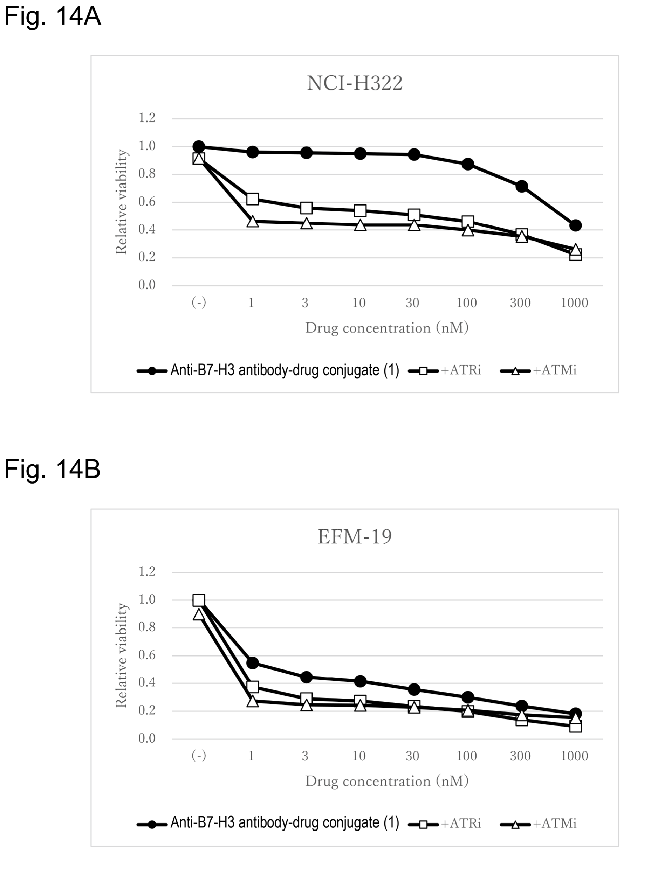

[Figure 14A] Figure 14A is a diagram showing the cell growth inhibitory effects of the anti-B7-H3 antibody-drug conjugate (1) as a single agent, and the combination treatment of the anti-B7-H3 antibody-drug conjugate (1) and ATRi (AZD6738), or ATMi (AZD1390), on human lung cancer cell NCI-H322. Black circles represent groups treated with the anti-B7-H3 antibody-drug conjugate (1) at each concentration as a single agent, white squares represent groups treated with the anti-B7-H3 antibody-drug conjugate (1) at each concentration and AZD6738 (final concentration 300 nM) as combination agents, and white triangles represent groups treated with the anti-B7-H3 antibody-drug conjugate (1) at each concentration and AZD1390 (final concentration 100 nM) as combination agents. Additionally, (-) represents groups untreated with the anti-B7-H3 antibody-drug conjugate (1).
[Figure 14B] Figure 14B is a diagram showing the cell growth inhibitory effects of the anti-B7-H3 antibody-drug conjugate (1) as a single agent, and the combination treatment of the anti-B7-H3 antibody-drug conjugate (1) and ATRi (AZD6738), or ATMi (AZD1390), on human breast cancer cell line EFM-19. Black circles represent groups treated with the anti-B7-H3 antibody-drug conjugate (1) at each concentration as a single agent, white squares represent groups treated with the anti-B7-H3 antibody-drug conjugate (1) at each concentration and AZD6738 (final concentration 300 nM) as combination agents, and white triangles represent groups treated with the anti-B7-H3 antibody-drug conjugate (1) at each concentration and AZD1390 (final concentration 100 nM) as combination agents. Additionally, (-) represents groups untreated with the anti-B7-H3 antibody-drug conjugate (1).
[Figure 15] Figure 15 is a diagram showing in vivo antitumor activities of Vehicle control; ATRi (AZD6738) as a single agent; anti-B7-H3 antibody-drug conjugate (1) as a single agent; and the combination treatment of anti-B7-H3 antibody-drug conjugate (1) and ATRi (AZD6738), respectively.
[Figure 16] Figure 16 is a diagram showing in vivo antitumor activities of Vehicle control; ATMi (AZD1390) as a single agent; anti-B7-H3 antibody-drug conjugate (1) as a single agent; and the combination treatment of anti-B7-H3

antibody-drug conjugate (1) and ATMi (AZD1390), respectively.

[Description of Embodiments]

Hereinafter, preferred modes for carrying out the present invention are described. The embodiments described below are given merely for illustrating one example of a typical embodiment of the present invention and are not intended to limit the scope of the present invention.

1. Definition

In the present Description, the term "cancer" is used to have the same meaning as that of the term "tumor".

In the present Description, the term "gene" includes not only DNA but also mRNA, cDNA thereof and cRNA thereof.

In the present Description, "CDR" means Complementarity Determining Region (CDR). It is known that a heavy chain and a light chain of an antibody molecular have CDRs at three locations, respectively. A CDR is also called a hypervariable region, which is a highly variable site of the primary structure in the variable regions of a heavy chain and a light chain of an antibody, and present in three locations separately in the primary structure of a polypeptide chain of a heavy chain and a light chain. In the present Description, for the CDR of an antibody, the CDRs of a heavy chain is written as CDRH1, CDRH2, and CDRH3 from the amino terminal side of the heavy chain amino acid sequence, and the CDRs of a light chain is written as CDRL1, CDRL2, and CDRL3 from the amino terminal side of the light chain amino acid sequence. These regions are close to each other on the steric structure and determine the specificity of the antibody to an antigen to which the antibody binds.

In the present Description, "approximately" refers to values that range from plus or minus 10%, 8%, 6%, 5%, 4%, 3%, 2% or 1%, respectively, of a reference value. Preferably, "approximately" refers to a range from plus or minus 10%, 5% or 1%, respectively, of a reference value.

2. Anti-B7-H3 antibody-drug conjugate

The anti-B7-H3 antibody-drug conjugate used in the present invention is known in the art. Examples of such anti-B7-H3 antibody-drug conjugates include, but are not limited to, vobramitamab duocarmazine (MGC018), mirzotamab clezutoclax (ABBV-155), BAT8009, HS-20093, MHB088C, 7MW3711, and DB-1311. Anti-B7-H3 antibody-drug conjugates are also described in WO2014/057687, WO2023/061457, US11,685,742B2, US20210347894A, US20220233708A, WO2024/022372, WO2022/117040, WO2024/037503, WO2024/037503, and WO2023/241663, all of which are incorporated herein by reference.

In some embodiments, the anti-B7-H3 antibody-drug conjugate used in the present invention is an anti-B7-H3 antibody-drug conjugate in which a drug-linker represented by the following formula:

[Formula 7]

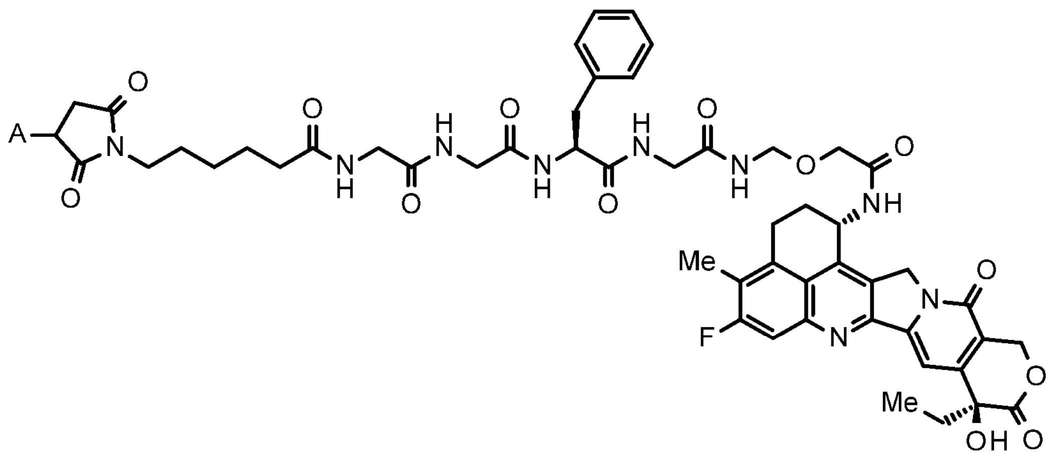

wherein A represents a connecting position to an anti-B7-H3 antibody, and the anti-B7-H3 antibody are conjugated via a thioether bond.

In the present invention, the partial structure consisting of a linker and a drug in the anti-B7-H3 antibody-drug conjugate is referred to as a "drug-linker". The drug-linker is connected to a thiol group (in other words, the sulfur atom of a cysteine residue) formed at an interchain disulfide bond site (two sites between heavy chains, and two sites between a heavy chain and a light chain) in the antibody.

In some embodiments, the drug-linker of the present invention includes exatecan (IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino [1,2-b]quinolin-10,13-dione, (also expressed as chemical name: (1S,9S)-1-amino-9-ethyl-5-fluoro-2,3-dihydro-9-hydroxy-4-methyl-1H,12H-benzo[de]pyrano[3',4':6,7]indolizino[1,2-b]quinolin-10,13(9H,15H)-dione)), which is a topoisomerase I inhibitor, as a component. Exatecan is a camptothecin derivative having an antitumor effect, represented by the following formula:

[Formula 8]

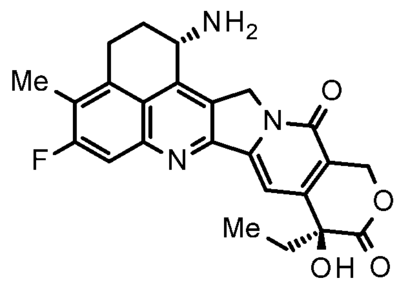

In some embodiments, the anti-B7-H3 antibody-drug conjugate used in the present invention can also be represented by the following formula:

[Formula 9]

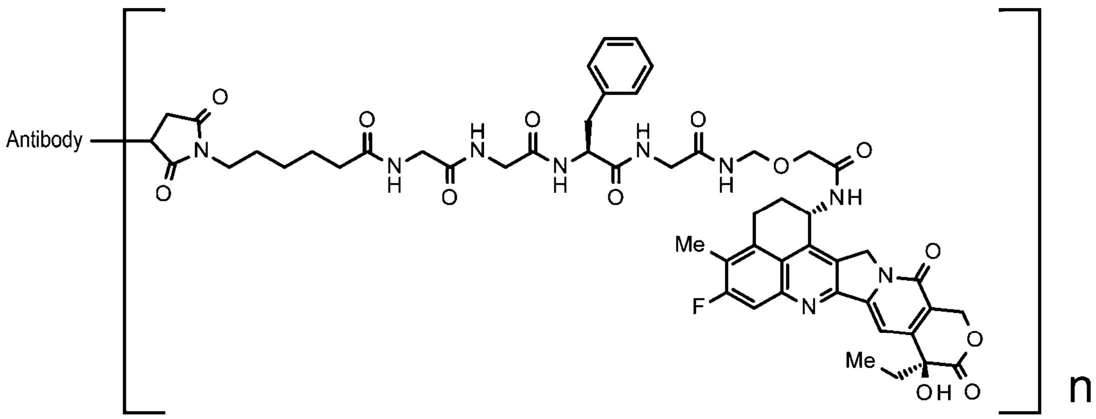

wherein the drug-linker is conjugated to an anti-B7-H3 antibody via a thioether bond. The meaning of n is the same as the so-called average number of conjugated drug molecules (DAR; Drug-to-Antibody Ratio), and indicates the average number of units of the drug-linker conjugated per antibody molecule. In the present invention, the "average number of conjugated drug molecules" is also called the drug-to-antibody ratio (DAR), and refers to the average number of drug molecules (or units of a drug-linker) that are conjugated per antibody molecule in a composition of antibody-drug conjugates.

In some embodiments, after migrating into cancer cells, the anti-B7-H3 antibody-drug conjugate used in the present invention releases the compound represented by the following formula:

[Formula 10]

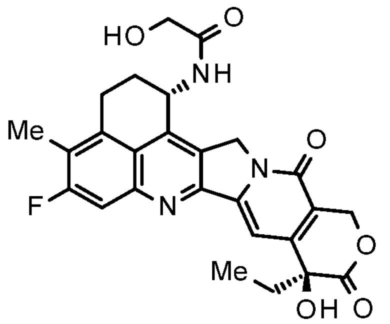

and thereby exerts an antitumor effect.

The aforementioned compound is inferred to be the original source of the antitumor activity of the anti-B7-H3 antibody-drug conjugate used in the present invention, and is presumed to have a topoisomerase I inhibitory effect (Ogitani Y. et al., Clinical Cancer Research, 2016, Oct 15; 22(20):5097-5108, Epub 2016 Mar 29).

In some embodiments, the anti-B7-H3 antibody-drug conjugate used in the present invention is ifinatamab deruxtecan.

In some embodiments, the anti-B7-H3 antibody-drug conjugate used in the present invention is also presumed to have a bystander effect (Ogitani Y. et al., Cancer Science (2016) 107, 1039-1046).

The bystander effect is considered to be exerted through a process such that the anti-B7-H3 antibody-drug conjugate used in the present invention is internalized in cancer cells expressing the target and the aforementioned compound is released and then exerts an antitumor effect also on nearby cancer cells not expressing the target.

3. Anti-B7-H3 antibody

B7-H3, one of the B7 family members expressed in antigen-presenting cells as a co-stimulator, is considered to act on receptors on T cells and enhance or suppress immune effect.

B7-H3 is a single transmembrane protein and has two variants. B7-H3 variant 1 (4Ig-B7-H3) contains a V-like or C-like Ig domain at two sites, respectively, and B7-H3 variant 2 (2Ig-B7-H3) contains a V-like or C-like Ig domain at one site, respectively.

As for B7-H3 to be used in the invention, B7-H3 can be directly purified from B7-H3-expressing cells of a human or a non-human mammal (such as a rat or a mouse) and used, or a cell membrane fraction of the above-described cells can be prepared and used. Further, B7-H3 can be obtained by in vitro synthesis thereof or production thereof in a host cell through genetic engineering. In the genetic engineering, specifically, after B7-H3 cDNA is integrated into a vector capable of expressing B7-H3 cDNA, B7-H3 can be obtained by synthesizing it in a solution containing an enzyme, a substrate and translation, or by expressing B7-H3 in another prokaryotic or eucaryotic transformed host cell.

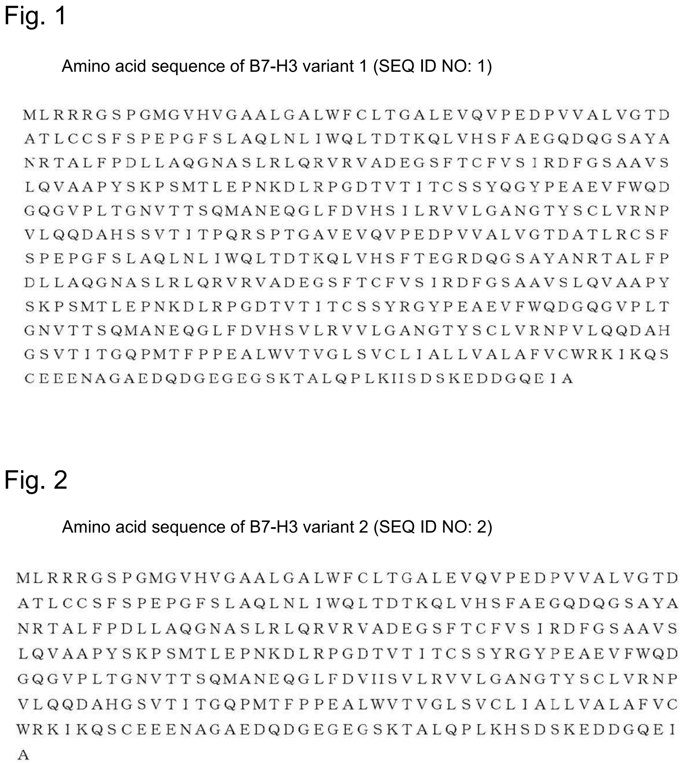

The amino acid sequence of the open reading frame (ORF) of the human B7-H3 variant 1 gene is represented by SEQ ID NO: 1 of the Sequence Listing. The sequence of SEQ ID NO: 1 is shown in Figure 1.

The amino acid sequence of the ORF of the human B7-H3 variant 2 gene is represented by SEQ ID NO: 2 of the Sequence Listing. The sequence of SEQ ID NO: 2 is shown in Figure 2.

In each of the above amino acid sequences of B7-H3, proteins consisting of an amino acid sequence in which one or several amino acids are substituted, deleted and/or added and having equivalent biological activities to the above protein are also included in B7-H3.

Mature human B7-H3 variant 1 from which a signal sequence is removed corresponds to the amino acid sequence consisting of amino acid residues 27 to 534 of the amino acid sequence represented by SEQ ID NO: 1. Mature human B7-H3 variant 2 from which a signal sequence is removed corresponds to the amino acid sequence consisting of amino acid residues 27 to 316 of the amino acid sequence represented by SEQ ID NO: 2.

In some embodiments, the anti-B7-H3 antibody in the anti-B7-H3 antibody-drug conjugate used in the present invention may be derived from any species, but is preferably an antibody derived from a human, a rat, a mouse, or a rabbit. In cases when the antibody is derived from species other than human species, it is preferably chimerized or humanized using a well-known technique. The antibody of the present invention may be a polyclonal antibody or a monoclonal antibody and is preferably a monoclonal antibody.

The anti-B7-H3 antibody in the anti-B7-H3 antibody-drug conjugate used in the present invention is an antibody preferably having the characteristic of being able to target cancer cells, and is preferably an antibody possessing the property of being able to recognize a cancer cell, the property of being able to bind to a cancer cell, the property of being

incorporated and internalized in a cancer cell, and/or cytocidal activity against cancer cells and/or the like.

The binding activity of the antibody against cancer cells can be confirmed using flow cytometry. The internalization of the antibody into cancer cells can be confirmed using (1) an assay visualizing an antibody incorporated into cells under a fluorescence microscope using a secondary antibody (fluorescently labeled) which binds to the therapeutic antibody (Cell Death and Differentiation (2008) 15, 751-761), (2) an assay measuring a fluorescence intensity incorporated into cells using a secondary antibody (fluorescently labeled) which binds to the therapeutic antibody (Molecular Biology of the Cell, Vol. 15, 5268-5282, December 2004), or (3) a Mab-ZAP assay using an immunotoxin which binds to the therapeutic antibody wherein the toxin is released upon incorporation into cells to inhibit cell growth (Bio Techniques 28: 162-165, January 2000). As the immunotoxin, a recombinant complex protein of a diphtheria toxin catalytic domain and protein G may be used.

The antitumor activity of the antibody can be confirmed in vitro by determining inhibitory activity against cell growth. For example, a cancer cell line overexpressing a target protein for the antibody is cultured, and the antibody is added at varying concentrations into the culture system in order to determine inhibitory activity against focus formation, colony formation, and spheroid growth. The antitumor activity can be confirmed in vivo, for example, by administering the antibody to nude mice inoculated with a cancer cell line highly expressing the target protein, and determining changes in the cancer cells.

Since the compound conjugated in the anti-B7-H3 antibody-drug conjugate exerts an antitumor effect, it is preferred but not essential that the anti-B7-H3 antibody itself should have an antitumor effect. For the purpose of specifically and selectively exerting the cytotoxic activity of the antitumor compound against cancer cells, it is important and also preferred that the antibody should have the property of being internalized and migrating into cancer cells.

The anti-B7-H3 antibody in the anti-B7-H3 antibody-drug conjugate used in the present invention can be obtained by a procedure known in the art. For example, the antibody of the present invention can be obtained using a method usually carried out in the art, which involves immunizing animals with an antigenic polypeptide, and collecting and purifying antibodies produced in vivo. The origin of the antigen is not limited to humans, and the animals may be immunized with an antigen derived from a non-human animal such as a mouse, a rat and the like. In this case, the cross-reactivity of antibodies binding to the obtained heterologous antigen with human antigens can be tested to screen for an antibody applicable to a human disease.

Alternatively, antibody-producing cells which produce antibodies against the antigen can be fused with myeloma cells according to a method known in the art (e.g., Kohler and Milstein, Nature (1975) 256, p. 495-497; Kennet, R. ed., Monoclonal Antibodies, p. 365-367, Plenum Press, N.Y. (1980)) to establish hybridomas, from which monoclonal antibodies can in turn be obtained.

The antigen can be obtained by genetically engineering host cells to produce a gene encoding the antigenic protein. Specifically, vectors that permit expression of the antigen gene are prepared and transferred to host cells so that the gene is expressed. The antigen thus expressed can be purified. The antibody can also be obtained by a method of immunizing animals with the above-described genetically engineered antigen-expressing cells or a cell line expressing the antigen.

The anti-B7-H3 antibody in the anti-B7-H3 antibody-drug conjugate used in the present invention is preferably a recombinant antibody obtained by artificial modification for the purpose of decreasing heterologous antigenicity to humans such as a chimeric antibody or a humanized antibody, or is preferably an antibody having only the gene sequence of an antibody derived from a human, that is, a human antibody. These antibodies can be produced using a known method.

An example of a chimeric antibody is an antibody in which antibody variable and constant regions are derived from different species, for example, a chimeric antibody in which a mouse- or rat-derived antibody variable region is connected to a human-derived antibody constant region (Proc. Natl. Acad. Sci. USA, 81, 6851-6855, (1984)).

Examples of a humanized antibody are an antibody obtained by integrating only the complementarity determining region (CDR) of a heterologous antibody into a human-derived antibody (Nature (1986) 321, pp. 522-525), an antibody obtained by grafting a part of the amino acid residues of the framework of a heterologous antibody as well as the CDR sequence of the heterologous antibody to a human antibody by a CDR-grafting method (WO 90/07861), and an antibody humanized using a gene conversion mutagenesis strategy (U.S. Patent No. 5821337).

An example of a human antibody is an antibody generated by using a human antibody-producing mouse having a human chromosome fragment including genes of a heavy chain and a light chain of a human antibody (see Tomizuka, K. et al., Nature Genetics (1997) 16, p.133-143; Kuroiwa, Y. et. al., Nucl. Acids Res. (1998) 26, p.3447-3448; Yoshida, H. et. al., Animal Cell Technology: Basic and Applied Aspects vol.10, p.69-73 (Kitagawa, Y., Matsuda, T. and Iijima, S. eds.), Kluwer Academic Publishers, 1999; Tomizuka, K. et. al., Proc. Natl. Acad. Sci. USA (2000) 97, p.722-727, etc.). An alternative example is an antibody obtained by phage display, the antibody being selected from a human antibody library (see Wormstone, I. M. et. al, Investigative Ophthalmology & Visual Science. (2002)43 (7), p.2301-2308; Carmen, S. et. al., Briefings in Functional Genomics and Proteomics (2002), 1(2), p.189-203; Siriwardena, D. et. al., Ophthalmology (2002) 109(3), p.427-431, etc.).

In some embodiments, in the anti-B7-H3 antibody in the anti-B7-H3 antibody-drug conjugate used in present

invention, modified variants of the antibody are also included. The modified variant refers to a variant obtained by subjecting the antibody according to the present invention to chemical or biological modification. Examples of the chemically modified variant include variants including a linkage of a chemical moiety to an amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N-terminus by being expressed in a prokaryotic host cell. Further, an antibody labeled so as to enable the detection or isolation of the antibody or an antigen according to the present invention, for example, an enzyme-labeled antibody, a fluorescence-labeled antibody and an affinity-labeled antibody are also included in the meaning of the modified variant. Such a modified variant of the antibody according to the present invention is useful for improving the stability and blood retention of the antibody, reducing the antigenicity thereof, detecting or isolating an antibody or an antigen, and so on.

Further, in some embodiments, by regulating the modification of a glycan which is linked to the antibody according to the present invention (glycosylation, defucosylation, etc.), it is possible to enhance antibody-dependent cellular cytotoxic activity. As the technique for regulating the modification of a glycan of antibodies, WO 99/54342, WO 00/61739, WO 02/31140, WO 2007/133855, and WO 2013/120066 etc. are known. However, the technique is not limited thereto. In the antibody according to the present invention, antibodies in which the modification of a glycan is regulated are also included.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (complement activation, antibody-dependent cellular cytotoxicity, etc.) of the antibody.

Therefore, in some embodiments, as the anti-B7-H3 antibody used in the present invention, antibodies subjected to such modification and various functional fragments of the antibody are also included. Furthermore, deletion variants in which one or two amino acids have been deleted at the carboxyl terminus of the heavy chain, variants obtained by amidation of the deletion variants (for example, a heavy chain in which the carboxyl terminal proline residue has been amidated), and the like are also included. The type of deletion variant having a deletion at the carboxyl terminus of the heavy chain of the antibody used in the present invention is not limited to the above variants so long as the antigen-binding affinity and the effector function are conserved. The two heavy chains constituting the antibody according to the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions; however, it is preferred that, in an antibody of the present invention, one amino acid residue at the carboxyl terminus has been deleted from each of the two heavy chains of the antibody.

As used herein, the term "functional fragment" of an antibody, also called "antigen-binding fragment of an antibody" in some embodiments, is used to mean a partial fragment of the antibody having binding activity against an antigen, and includes, but is not limited to, Fab, F(ab')2, scFv, a diabody, a linear antibody and a multispecific antibody formed from antibody fragments, and the like. In some embodiments, Fab', which is a monovalent fragment of the antibody variable regions obtained by treating F(ab')2 under reducing conditions, is also included in the antigen-binding fragment of an antibody. The antigen-binding fragment of an antibody is not limited to these molecules, so long as the antigen-binding fragment has antigen-binding ability. These antigen-binding fragments include not only those obtained by treating a full-length molecule of an antibody protein with an appropriate enzyme, but proteins produced in appropriate host cells using a genetically engineered antibody gene.

Isotypes of the antibody according to the present invention may be, for example, IgG (IgG1, IgG2, IgG3, IgG4), and preferably IgG1 or IgG2.

In the present invention, the term "anti-B7-H3 antibody" refers to an antibody which binds specifically to B7-H3 (B cell antigen #7 homolog 3; PD-L3; CD276), and preferably has an activity of internalization in B7-H3-expressing cells by binding to B7-H3.

Examples of the anti-B7-H3 antibody include any combinations of a heavy chain comprising a heavy chain variable region consisting of any one of (1) the amino acid sequence consisting of amino acid residues 20 to 141 of SEQ ID NO: 3, 5, 6 or 7 of the Sequence Listing, (2) an amino acid sequence having at least 95% or more homology with the amino acid sequence of the above (1), and (3) an amino acid sequence of the above (1) in which one or several amino acids are deleted, substituted or added, and a light chain comprising a light chain variable region consisting of any one of (4) the amino acid sequence consisting of amino acid residues 21 to 128 of SEQ ID NO: 4, 8, 9, 10, 11, 12 or 13, (5) an amino acid sequence having at least 95% or more homology with the amino acid sequence of the above (4), and (6) an amino acid sequence of the above (4) in which one or several amino acids are deleted, substituted or added, and preferably, M30-H1-L4 (International Publication No. WO 2014/057687). The term "several" in the present specification means 1 to 10, 1 to 9, 1 to 8, 1 to 7, 1 to 6, 1 to 5, 1 to 4, 1 to 3, or 1 or 2.

For the substitution of amino acids in the present specification, conserved amino acid substitution is preferred. The conserved amino acid substitution is a replacement which occurs within amino acid groups associated with amino acid side chains. Preferable amino acid groups are as follows: acidic group = aspartic acid, glutamic acid; basic group = lysine, arginine, histidine; non-polar group = alanine, valine, leucine, isoleucine, proline, phenylalanine, methionine, tryptophan; and non- charged, polar family = glycine, asparagine, glutamine, cysteine, serine, threonine, and tyrosine. Other preferable amino acid groups are as follows: aliphatic hydroxy group = serine and threonine; amide-containing group = asparagine and glutamine; aliphatic group = alanine, valine, leucine, and isoleucine; and aromatic group = phenylalanine, tryptophan, and tyrosine. Such an amino acid substitution is preferably carried out in a range which does not decrease the properties of the substance having the original amino acid sequence.

Examples of the antibody having a preferable combination of the above heavy chain and light chain include an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, or an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

Examples of the antibody having a more preferable combination include an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence

consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

Further, other preferable combinations can include an antibody consisting of a heavy chain consisting of an amino acid sequence represented by SEQ ID NO: 3 and a light chain consisting of an amino acid sequence represented by SEQ ID NO: 8, an antibody consisting of a heavy chain consisting of an amino acid sequence represented by SEQ ID NO: 3 and a light chain consisting of an amino acid sequence represented by SEQ ID NO: 9, an antibody consisting of a heavy chain consisting of an amino acid sequence represented by SEQ ID NO: 3 and a light chain consisting of an amino acid sequence represented by SEQ ID NO: 10, an antibody consisting of a heavy chain consisting of an amino acid sequence represented by SEQ ID NO: 3 and a light chain consisting of an amino acid sequence represented by SEQ ID NO: 4, an antibody consisting of a heavy chain consisting of an amino acid sequence represented by SEQ ID NO: 3 and a light chain consisting of an amino acid sequence represented by SEQ ID NO: 11, an antibody consisting of a heavy chain consisting of an amino acid sequence represented by SEQ ID NO: 3 and a light chain consisting of an amino acid sequence represented by SEQ ID NO: 12, an antibody consisting of a heavy chain consisting of an amino acid sequence represented by SEQ ID NO: 3 and a light chain consisting of an amino acid sequence represented by SEQ ID NO: 13, an antibody consisting of a heavy chain consisting of an amino acid sequence represented by SEQ ID NO: 7 and a light chain consisting of an amino acid sequence represented by SEQ ID NO: 8, an antibody consisting of a heavy chain consisting of an amino acid sequence represented by SEQ ID NO: 7 and a light chain consisting of an amino acid sequence represented by SEQ ID NO: 9, an antibody consisting of a heavy chain consisting of an amino acid sequence represented by SEQ ID NO: 7 and a light chain consisting of an amino acid sequence represented by SEQ ID NO: 10, or an antibody consisting of a heavy chain consisting of an amino acid sequence represented by SEQ ID NO: 7 and a light chain consisting of an amino acid sequence represented by SEQ ID NO: 4.

By combining sequences having high homologies with the above heavy chain amino acid sequences and light chain amino acid sequences, it is possible to select antibodies having a cellular cytotoxic activity equivalent to each of the aforementioned antibodies. Such a homology is typically 80% or more homology, preferably 90% or more homology, more preferably 95% or more homology, and most preferably 99% or more homology. Alternatively, by combining amino acid sequences in which one to several amino acid residues are substituted, deleted or added in amino acid sequences of the heavy chain or light chain, it is possible to select antibodies having a cellular cytotoxic activity equivalent to each of the aforementioned antibodies.

The homology between two types of amino acid sequences can be determined by using the default parameter of Blast algorithm version 2.2.2 (Altschul, Stephen F., Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res.25:3389-3402). Blast algorithm can also be used by accessing www.ncbi.nlm.nih.gov/blast on the internet.

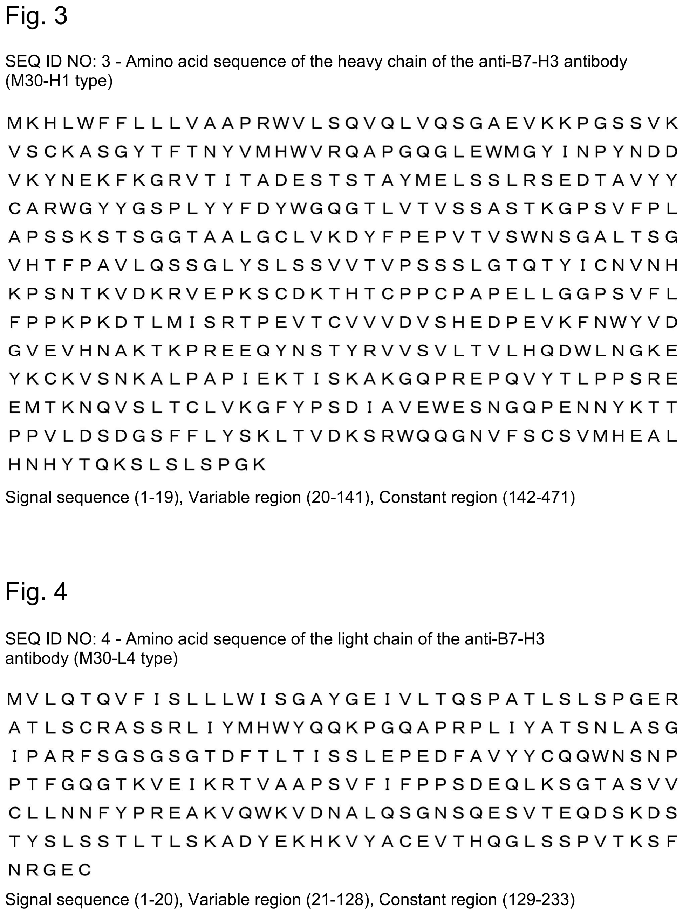

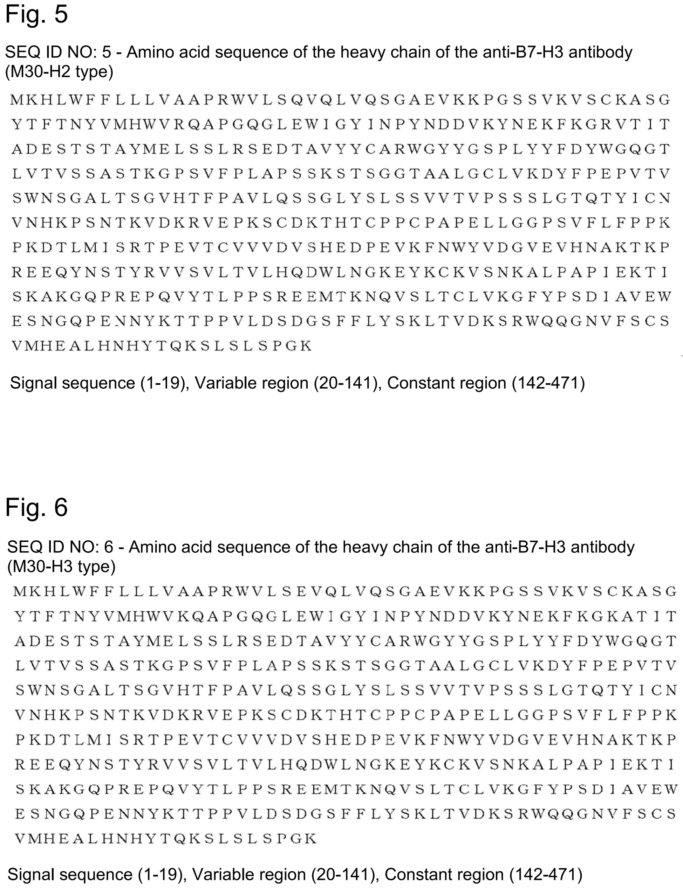

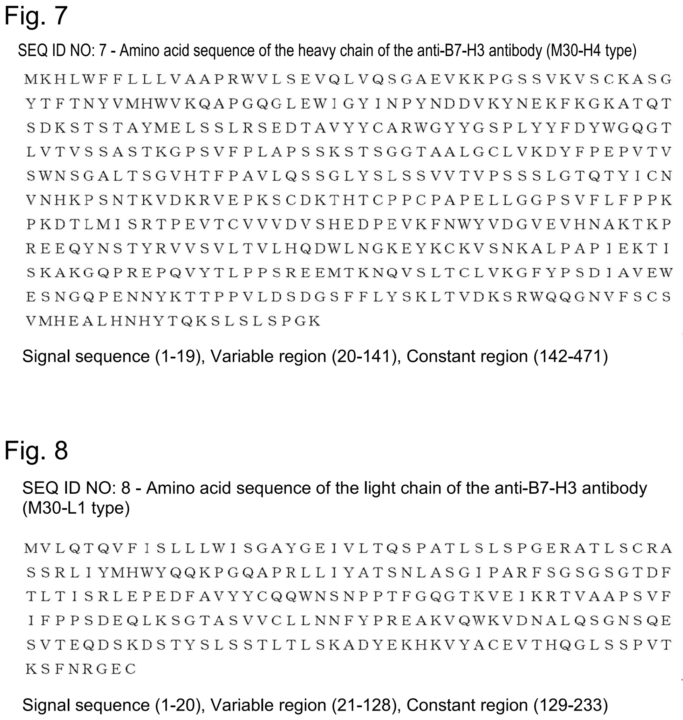

In the heavy chain amino acid sequence represented by SEQ ID NO: 3, 5, 6 or 7 of the Sequence Listing, the amino acid sequence consisting of amino acid residues 1 to 19 is a signal sequence, the amino acid sequence consisting of amino acid residues 20 to 141 is a variable region, and the amino acid sequence consisting of amino acid residues 142 to 471 is a constant region. The sequence of SEQ ID NO: 3 is shown in Figure 3, the sequence of SEQ ID NO: 5 is shown in Figure 5, the sequence of SEQ ID NO: 6 is shown in Figure 6, and the sequence of SEQ ID NO: 7 is shown in Figure 7, respectively.

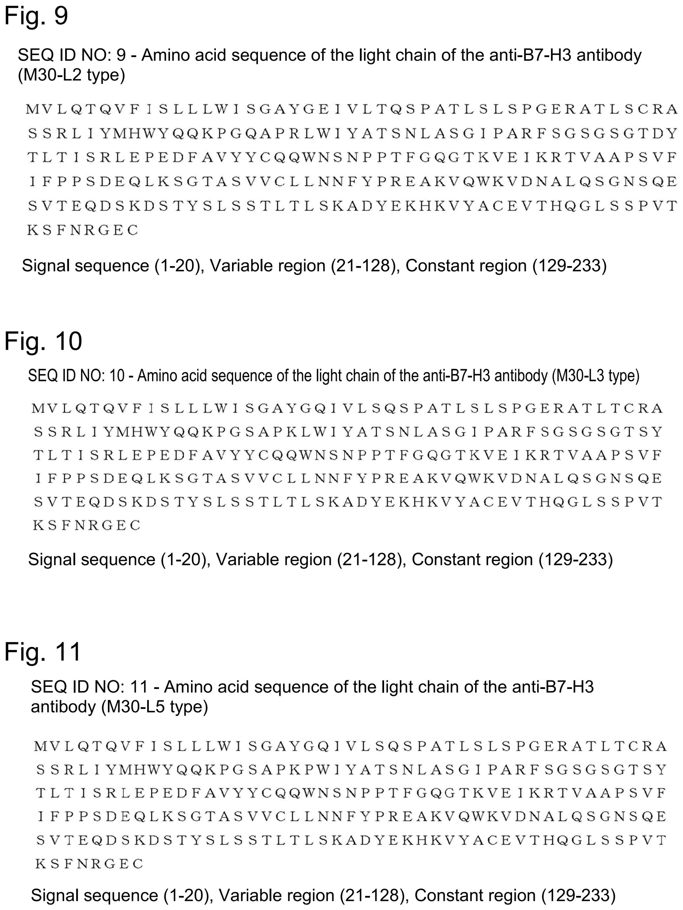

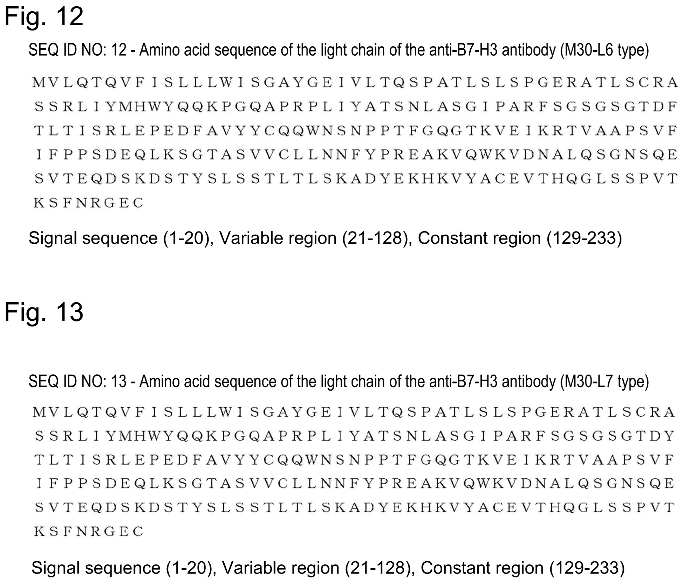

In the light chain amino acid sequence represented by SEQ ID NO: 4, 8, 9, 10, 11, 12 or 13 of the Sequence Listing, the amino acid sequence consisting of amino acid residues 1 to 20 is a signal sequence, the amino acid sequence consisting of amino acid residues 21 to 128 is a variable region, and the amino acid sequence consisting of amino acid residues 129 to 233 is a constant region. The sequence of SEQ ID NO: 4 is shown in Figure 4, the sequence of SEQ ID NO: 8 is shown in Figure 8 the sequence of SEQ ID NO: 9 is shown in Figure 9, the sequence of SEQ ID NO: 10 is shown in Figure 10, the sequence of SEQ ID NO: 11 is shown in Figure 11, the sequence of SEQ ID NO: 12 is shown in Figure 12, and the sequence of SEQ ID NO: 13 is shown in Figure 13, respectively.

4. Production of anti-B7-H3 antibody-drug conjugate

A drug-linker intermediate for use in the production of the anti-B7-H3 antibody-drug conjugate used in the present

invention is represented by the following formula.

[Formula 11]

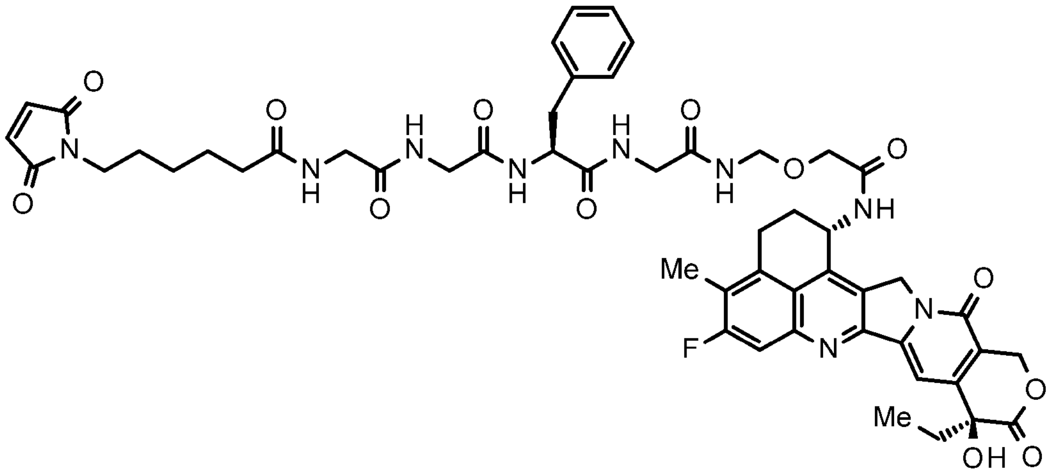

The drug-linker intermediate can be expressed as the chemical name N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide, and can be produced with reference to descriptions in International Publication No. WO 2014/057687, International Publication No. WO 2015/098099, International Publication No. WO 2015/115091, International Publication No. WO 2015/155998, International Publication No. WO 2019/044947, and so on.

The anti-B7-H3 antibody-drug conjugate used in the present invention can be produced by reacting the above-described drug-linker intermediate and an anti-B7-H3 antibody having a thiol group (alternatively referred to as a sulfhydryl group).

The antibody having a sulfhydryl group can be obtained by a method well known in the art (Hermanson, G. T, Bioconjugate Techniques, pp. 56-136, pp. 456-493, Academic Press (1996)). For example, by using 0.3 to 3 molar equivalents of a reducing agent such as tris(2-carboxyethyl)phosphine hydrochloride (TCEP) per interchain disulfide within the antibody and reacting with the antibody in a buffer solution containing a chelating agent such as ethylenediamine tetraacetic acid (EDTA), an antibody having a sulfhydryl group with partially or completely reduced interchain disulfides within the antibody can be obtained.

Further, by using 2 to 20 molar equivalents of the drug-linker intermediate per the antibody having a sulfhydryl group, an antibody-drug conjugate in which 2 to 8 drug molecules are conjugated per antibody molecule can be produced.

The average number of conjugated drug molecules per antibody molecule of the antibody-drug conjugate produced can be determined, for example, by a method of calculation based on measurement of UV absorbance by the anti-B7-H3 antibody-drug conjugate and the conjugation precursor thereof at two wavelengths of 280 nm and 370 nm (UV method), or a method of calculation based on quantification through HPLC measurement of fragments obtained by treating the antibody-drug conjugate with a reducing agent (HPLC method).

Conjugation between the antibody and the drug-linker intermediate, and calculation of the average number of conjugated drug molecules per antibody molecule of the antibody-drug conjugate, can be performed with reference to descriptions in International Publication No. WO 2014/057687, International Publication No. WO 2017/002776, and so on.

In the present invention, the term "anti-B7-H3 antibody-drug conjugate" refers to an antibody-drug conjugate such that the antibody in the antibody-drug conjugate according to the present invention is an anti-B7-H3 antibody.

The anti-B7-H3 antibody is preferably an antibody comprising (a heavy chain variable region comprising) CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3, and (a light chain variable region comprising) CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4,

more preferably an antibody comprising a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 4, and

even more preferably an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, or a variant of the antibody in which a lysine residue at the carboxyl terminus of (both) the heavy chains is deleted.

In some embodiments, the average number of units of the drug-linker conjugated per antibody molecule in the anti-B7-H3 antibody-drug conjugate is preferably 2 to 8, more preferably 3 to 5, even more preferably 3.5 to 4.5, and even more preferably approximately 4. In the present invention, the term "approximately 4" is preferably 3.8 to 4.2, more preferably 3.9 to 4.1, and even more preferably 4. In other embodiments, the number of the drug or the drug-linker conjugated per antibody molecule in the anti-B7-H3 antibody-drug conjugate is an integer in the range from preferably 2 to 8, more preferably selected from 2, 4, 6, or 8, and most preferably 4.

The anti-B7-H3 antibody-drug conjugate used in the present invention can be produced with reference to descriptions in International Publication No. WO 2014/057687, International Publication No. WO 2017/002776 and so on.

5. ATR inhibitor and ATM inhibitor

In the present invention, "ATR inhibitor" refers to an agent that inhibits the kinase activity of Ataxia- telangiectacia mutated and Rad3-related (ATR).

The ATR inhibitor in the present invention can have the inhibitory effect on kinases other than ATR, but is preferably an agent that selectively inhibits ATR kinase activity.

The ATR inhibitor in the present invention is not limited so long as an agent has the above characteristic, but preferably examples include AZD6738, BAY-1895344 (CAS number 1876467-74-1) and ETP-46464 (CAS number 1345675-02-6), and pharmaceutically acceptable salts thereof.

The ATR inhibitor used in the present invention can be produced according to a known method, and for example, AZD6738 can be obtained according to WO2011/154737.

In the present invention, "ATM inhibitor" refers to an agent that inhibits the kinase activity of Ataxia-telangiectacia mutated (ATM).

The ATM inhibitor in the present invention can have an inhibitory effect on kinases other than ATM, but is preferably an agent that selectively inhibits ATM kinase activity.

The ATM inhibitor in the present invention is not limited so long as an agent has the above characteristic, but preferably examples include AZD1390 (WO2017/046216: Example 2), AZD0156 (WO2015/170081: Example 1), KU-55933 (CAS number 587871-26-9; WO2003070726A1), KU-60019 (CAS number 925701-49-1), KU-59403 (WO2005016919), CP466722 (CAS number 1080622-86-1), and NVP-BEZ235 (dactolisib; CAS number 915019-65-7; WO2006/122806), and pharmaceutically acceptable salts thereof.

The ATM inhibitor used in the present invention can be produced according to a known method, and for example, AZD1390 and AZD0156 can be obtained according to WO2017/046216 and WO2015/170081.

The ATR inhibitor or ATM inhibitor used in the present invention are not limited so long as agents have the above characteristic, but can be a low molecular weight compound, an antibody, a functional fragment of an antibody, a fusion protein, an immunoadhesin, a nucleic acid, an oligonucleotide, an aptamer, or a polypeptide, and preferably a low molecular weight compound.

The above low molecular weight compound is, in some embodiments, preferably an organic compound having a molecular weight of 1000 or less, more preferably a molecular weight of 900 or less, and even more preferably a molecular weight of 800 or less, even more preferably a molecular weight of 700 or less, even more preferably a molecular weight of 600 or less, even more preferably a molecular weight of 500 or less, even more preferably a molecular weight from 50 to 500, and even more preferably a molecular weight from 100 to 500.

The "pharmaceutically acceptable salt" of the ATR inhibitor or ATM inhibitor used in the present invention can be either an acid addition salt or a base addition salt. Examples of the acid addition salt include lower alkanesulfonates such as camsylate (camphorsulfonate), mesylate (methanesulfonate), trifluoromethanesulfonate, and ethanesulfonate; aryl sulfonates such as tosylate (p-toluenesulfonate), and benzenesulfonate; inorganic acid salts such as phosphate, nitrate, perchlorate, and sulfate; hydrohalic acid salts such as hydrochloride, hydrobromide, hydroiodide, and hydrofluoride; organic acid salts such as acetate, malate, fumarate, succinate, citrate, tartrate, oxalate, and maleate; and amino acid salts such as ornithinate, glutamate, and aspartate. Examples of the base addition salt include alkali metal salts such as sodium salt, potassium salt, and lithium salt; alkaline earth metal salts such as calcium salt, and magnesium salt; inorganic salts such as ammonium salt; organic amine salts such as dibenzylamine salt, morpholine salt, phenyl glycine alkyl ester salt, ethylene diamine salt, N-methylglucamine salt, diethylamine salt, triethylamine salt, cyclohexylamine salt, dicyclohexylamine salt, N,N'-dibenzyl ethylene diamine salt, diethanolamine salt, N-benzyl-N-(2-phenylethoxy)amine salt, piperazine salt, tetramethylammonium salt, and tris(hydroxymethyl)aminomethane salt; and amino acids salts such as arginine salt.

Additionally, the ATR inhibitor or ATM inhibitor used in the present invention, and pharmaceutically acceptable salts thereof, may also be present in the form of a solvate, and these solvates are also included in the ATR inhibitor or ATM inhibitor used in the present invention, and pharmaceutically acceptable salts thereof.

The ATR inhibitor or ATM inhibitor used in the present invention can include a therapeutically effective amount of the ATR inhibitor or ATM inhibitor, a pharmaceutically acceptable carrier, a diluent, a solubilizer, an emulsifier, a preservative, an auxiliary agent, and/or the like. The "pharmaceutically acceptable carrier" and the like can be appropriately selected from a wide range in view of the kind of target disease and the form of administration of an agent. The method of administration can be selected appropriately. For example, administration by injection can be performed, and local injection, intraperitoneal injection, selective intravenous injection, intravenous injection, subcutaneous injection, organ perfusion injection, or the like can be adopted. The injection solution can be formulated using a carrier composed of a salt solution, a glucose solution, or a mixture of brine and a glucose solution, various buffers, and the like. Alternatively, the injection solution can be formulated into the form of a powder and prepared to be an injection solution by being mixed with the solution carrier described above when used.

Other methods of administration can also be selected appropriately along with development of a formulation. For example, in the case of oral administration, an oral liquid, a powder, a pill, a capsule, a tablet, and the like can be applied. In the case of the oral liquid, such a liquid can be produced in the form of an oral liquid preparation such as a suspension and a syrup using water, sugars such as sucrose, sorbitol and fructose, glycols such as polyethylene glycol, oils such as sesame oil and soy oil, preservatives such as alkyl parahydroxybenzoate, flavors such as strawberry flavor and peppermint, and/or the like. The powder, pill, capsule, and tablet can be formulated using an excipient such as lactose, glucose, sucrose, and mannitol, a disintegrant such as starch and sodium alginate, a lubricant such as magnesium stearate and talc, a binder such as polyvinyl alcohol, hydroxypropyl cellulose and gelatine, a surfactant such as fatty acid ester, a plasticizer such as glycerin, and/or the like. The tablet and capsule are preferably in a unit dosage form for easy administration. For the production of the tablet and capsule, a solid manufacturing carrier is used.

6. Medicament

Hereinafter, the pharmaceutical composition and method of treatment comprising administering an anti-B7-H3 antibody-drug conjugate according to the present invention in combination with an ATR inhibitor or an ATM inhibitor are described.

In some embodiments, the present invention comprises a pharmaceutical composition comprising an anti-B7-H3 antibody-drug conjugate used in combination with an ATR inhibitor or an ATM inhibitor, or administered in combination with an ATR inhibitor or an ATM inhibitor.

In some embodiments, the pharmaceutical composition or the method of treatment of the present invention may comprise the anti-B7-H3 antibody-drug conjugate and the ATR inhibitor or ATM inhibitor as active components mutually in different formulations which are administered at the same time or different times, or may comprise the anti-B7-H3 antibody-drug conjugate and the ATR inhibitor or ATM inhibitor as active components in a single formulation which are administered.

In some embodiments, the pharmaceutical composition or the method of treatment of the present invention can also comprise administering a combination of 2 or more of the ATR inhibitor or the ATM inhibitor used in the present invention.

In some embodiments, the pharmaceutical composition or the method of treatment of the present invention can be used for treating cancer, preferably used for treating at least one selected from the group consisting of breast cancer, stomach cancer, colorectal cancer, lung cancer (for example, small cell lung cancer (SCLC) or non-small cell lung cancer (NSCLC)), esophageal cancer, head and neck cancer, gastroesophageal junction adenocarcinoma, biliary tract cancer, Paget’s disease, pancreatic cancer, ovarian cancer, uterine carcinosarcoma, urothelial cancer, prostate cancer (for example, castration-resistant prostate cancer (CRPC)), bladder cancer, gastrointestinal stromal tumor, cervical cancer, squamous cell cancer, peritoneal cancer, liver cancer, hepatocellular cancer, uterine cancer, kidney cancer, vulvar cancer, thyroid cancer, penile cancer, leukemia, malignant lymphoma, plasmacytoma, myeloma, glioblastoma multiforme, osteosarcoma, sarcoma, melanoma, and mesothelioma, more preferably can be used for treating at least one selected from the group consisting of breast cancer, lung cancer (for example, small cell lung cancer (SCLC) or non-small cell lung cancer (NSCLC)), esophageal cancer, head and neck cancer, prostate cancer (for example, castration-resistant prostate cancer (CRPC)), bladder cancer, squamous cell cancer, uterine cancer, sarcoma, and melanoma, even more preferably can be used for treating at least one selected from the group consisting of breast cancer, lung cancer (for example, small cell lung cancer (SCLC) or non-small cell lung cancer (NSCLC)), esophageal cancer, head and neck cancer, prostate cancer (for example, castration-resistant prostate cancer (CRPC)), and squamous cell cancer.

In other embodiments, the present invention relates to an agent for treating cancer used in combination with an ATR inhibitor or an ATM inhibitor, wherein a patient has been (already) treated with the ATR inhibitor or ATM inhibitor, and the agent comprises an anti-B7-H3 antibody-drug conjugate as an active component.

In other embodiments, the present invention relates to an agent for the treatment of cancer comprising an anti-B7-H3 antibody-drug conjugate as an active component, wherein the treatment comprises administering an ATR inhibitor or an ATM inhibitor.
In other embodiments, the present invention relates to an anti-B7-H3 antibody-drug conjugate for the treatment of cancer by being administered in combination with an ATR inhibitor or an ATM inhibitor.
In other embodiments, the present invention relates to use of an anti-B7-H3 antibody-drug conjugate in the manufacture of a medicament for the treatment of cancer, for use in combination with an ATR inhibitor or an ATM inhibitor.
In other embodiments, the present invention relates to a pharmaceutical combination of (i) an anti-B7-H3 antibody-drug conjugate and (ii) an ATR inhibitor or an ATM inhibitor.
In other embodiments, the present invention relates to a pharmaceutical composition comprising (i) an anti-B7-H3 antibody-drug conjugate and (ii) an ATR inhibitor or an ATM inhibitor for administration in combination.
In other embodiments, the present invention relates to a kit comprising (a) a first composition comprising an anti-B7-H3 antibody-drug conjugate and (b) a second composition comprising an ATR inhibitor or an ATM inhibitor.
The anti-B7-H3 antibody-drug conjugate used in the present invention can be preferably used when the expression of B7-H3 is confirmed in a cancer.
The presence or absence of B7-H3 tumor marker can be checked by, for example, collecting tumor tissues from a cancer patient and subjecting the formalin fixed paraffin embedded specimen (FFPE) to an examination at a gene product (protein) level, such as an immunohistochemistry (IHC) method, flow cytometry, a western blot method, or an examination at a gene transcription level, such as an in situ hybridization method (ISH), a quantitative PCR method (q-PCR), or a microarray analysis; alternatively, it can also be checked by collecting cell-free circulating tumor DNA (ctDNA) from a cancer patient and subjecting it to an examination which uses a method such as next-generation sequencing (NGS).
The pharmaceutical composition and the method of treatment of the present invention are preferably for use in mammals, and more preferably for use in humans.
An antitumor effect of the pharmaceutical composition and the method of treatment of the present invention can be confirmed by, for example, generating a model in which a cancer cell is transplanted into a test animal, applying the pharmaceutical composition and the method of treatment of the present invention, and measuring a decrease in a tumor volume and a life-extending effect. Then, a comparison is made with the antitumor effects gained from when each of the antibody-drug conjugate and the ATR inhibitor or ATM inhibitor used in the present invention is administered singly, whereby the effect of the combination of the antibody-drug conjugate and the ATR inhibitor or ATM inhibitor used in the present invention can be confirmed.
In addition, the antitumor effect of the pharmaceutical composition and the method of treatment of the present invention can be confirmed in a clinical study, with the Response Evaluation Criteria in Solid Tumors (RECIST) evaluation method, WHO's evaluation method, Macdonald's evaluation method, measurement of body weight, and other methods; and can be determined by indicators such as Complete response (CR), Partial response (PR), Progressive disease (PD), Objective response rate (ORR), Duration of response (DoR), Progression-free survival (PFS), and Overall survival (OS).
The foregoing methods can provide confirmation of superiority of the antitumor effect of the pharmaceutical composition and the method of treatment of the present invention compared to an existing pharmaceutical composition and a method of treatment for treating cancer.
In some embodiments, the pharmaceutical composition and the method of treatment of the present invention can retard growth of cancer cells, suppress their proliferation, and further can kill cancer cells. These effects can allow cancer patients to be free from symptoms caused by cancer or can achieve an improvement in the QOL of cancer patients and attain a therapeutic effect by sustaining the lives of the cancer patients. Even if the pharmaceutical composition and method of treatment do not accomplish the killing of cancer cells, they can achieve higher QOL of cancer patients while achieving longer-term survival, by inhibiting or controlling the growth of cancer cells.
In some embodiments, the pharmaceutical composition of the present invention can provide a therapeutic effect by being applied as a systemic therapy to patients or being applied locally to cancer tissues.
In some embodiments, the pharmaceutical composition of the present invention can be administered containing at least one pharmaceutically suitable ingredient. The pharmaceutically suitable ingredients can be appropriately selected and applied from formulation additives or the like that are generally used in the art, in view of the dosage, the administration concentration or the like of the antibody-drug conjugate and the ATR inhibitor or ATM inhibitor used in the present invention. For example, the antibody-drug conjugate used in the present invention can be administered as a pharmaceutical composition containing a buffer such as a histidine buffer, an excipient such as sucrose or trehalose, and a surfactant such as polysorbate 80 or 20. The pharmaceutical composition containing the anti-B7-H3 antibody-drug conjugate used in the present invention preferably can be used as an injection, more preferably can be used as an aqueous injection or a lyophilized injection, and even more preferably can be used as a lyophilized injection.
In the case that the pharmaceutical composition containing the anti-B7-H3 antibody-drug conjugate used in the present invention is an aqueous injection, it preferably can be diluted with a suitable diluent and then given as an intravenous infusion. For the diluent, examples can be a dextrose solution, physiological saline, and the like, and a

preferable example is a dextrose solution, and a more preferable example is a 5% dextrose solution.

In the case that the pharmaceutical composition containing the anti-B7-H3 antibody-drug conjugate used in the present invention is a lyophilized injection, it preferably can be dissolved in water for injection, subsequently a required amount can be diluted with a suitable diluent and then given as an intravenous infusion. For the diluent, examples can be a dextrose solution, physiological saline, and the like, and a preferable example is a dextrose solution, and a more preferable example is a 5% dextrose solution.

Examples of the administration route which may be used to administer the pharmaceutical composition of the present invention include intravenous, intradermal, subcutaneous, intramuscular and intraperitoneal routes, and preferably include an intravenous route.

In some embodiments, the anti-B7-H3 antibody-drug conjugate used in the present invention can be administered to a human at a dose of approximately 0.001 to 100 mg/kg once or multiple times with intervals of 1 to 180 days between each administration. The anti-B7-H3 antibody-drug conjugate can be administered preferably at a dose of 0.1 to 50 mg/kg, more preferably once every 1 to 4 weeks at a dose of 1 to 50 mg/kg, 1 to 30 mg/kg, 1 to 20 mg/kg, 1 to 15 mg/kg, 2 to 50 mg/kg, 2 to 30 mg/kg, 2 to 20 mg/kg, or 2 to 15 mg/kg, and preferably multiple times with one administration every 2 to 3 weeks. For example, the anti-B7-H3 antibody-drug conjugate can be administered once every 3 weeks at a dose of 0.8 mg/kg, 1.6 mg/kg, 3.2 mg/kg, 4.8 mg/kg, 6.4 mg/kg, 8.0 mg/kg, 10.0 mg/kg, 12.0 mg/kg, or 16.0 mg/kg, and preferably administered once every 3 weeks at a dose of 8.0 mg/kg, 10.0 mg/kg, or 12.0 mg/kg.

In some embodiments, the ATR inhibitor or ATM inhibitor according to the present invention can be administered orally to a human once to twice every 1 to 7 days, and can be preferably administered orally once daily, or twice daily. The ATR inhibitor or ATM inhibitor used in the present invention can be administered orally at a dose of 0.1 mg to 3000 mg, and preferably can be administered orally at a dose of 2.5 mg to 600 mg. The ATR inhibitor or ATM inhibitor according to the present invention can be given to a human as an intravenous infusion once every 1 to 180 days, and preferably can be given as an intravenous infusion once every week, 2 weeks, 3 weeks, or 4 weeks. The ATR inhibitor or ATM inhibitor used in the present invention can be given as an intravenous infusion at a dose of 0.1 mg to 3000 mg, and preferably can be given as an intravenous infusion at a dose of 10 mg to 100 mg.

The ATR inhibitor or ATM inhibitor used in the present invention is administered, as an example, by the method using the following dosage and administration, but the method is not limited thereto. For example, the ATR inhibitor or ATM inhibitor can be administered at a dose of 5 to 1000 mg once, twice, 3 times, 4 times, or 5 times daily.

For another dosage and administration, the ATR inhibitor or ATM inhibitor can be administered orally at a dose of 20 to 240 mg once daily.

For another dosage and administration, the ATR inhibitor or ATM inhibitor can be administered orally at a dose of 20, 40, 80, 120, 160, or 240 mg (preferably 40, 80, or 120 mg) once daily.

For another dosage and administration, the ATR inhibitor or ATM inhibitor can be administered orally at a dose of 120 mg once daily.

When compound AZD6738, or a pharmaceutically acceptable salt thereof, is used as the ATR inhibitor, the ATR inhibitor is preferably administered orally twice daily at a dose of 20 mg, 40 mg, 60 mg, 80 mg, 120 mg, 160 mg, 200 mg, or 240 mg.

In some embodiments, the pharmaceutical composition and the method of treatment of the present invention may further comprise cancer therapeutic agents other than the anti-B7-H3 antibody-drug conjugate according to the present invention and the ATR inhibitor or ATM inhibitor. The pharmaceutical composition and the method of treatment of the present invention can also be administered in combination with other cancer therapeutic agents, thereby enhancing the antitumor effect. Other cancer therapeutic agents used for this purpose may be administered to a subject simultaneously (here, a person skilled in the art would naturally understand that "simultaneously" may be or may not be at approximately the same time) with, separately from, or subsequently to the pharmaceutical composition of the present invention, and may be administered while varying the administration interval for each. The subject includes, but is not limited to, preferably a mammal and more preferably a human. Such cancer therapeutic agents are not limited so long as they are agents having antitumor activity, and may be for example at least one selected from the group consisting of irinotecan (CPT-11), cisplatin, carboplatin, oxaliplatin, fluorouracil (5-FU), gemcitabine, capecitabine, doxorubicin, epirubicin, cyclophosphamide, mitomycin C, tegafur-gimeracil-oteracil combination, panitumumab, bevacizumab, ramucirumab, regorafenib, trifluridine-tipiracil combination, gefitinib, erlotinib, afatinib, methotrexate, pemetrexed, tamoxifen, toremifene, fulvestrant, leuprorelin, goserelin, letrozole, anastrozole, progesterone formulation and lapatinib.

In some embodiments, the pharmaceutical composition and the method of treatment of the present invention can also be used in combination with radiotherapy. For example, a cancer patient may receive radiotherapy before and/or after receiving, or simultaneously with, the treatment by the pharmaceutical composition of the present invention.

In some embodiments, the pharmaceutical composition and the method of treatment of the present invention can also be used as an adjunctive chemotherapy in combination with a surgical procedure. The pharmaceutical composition of the present invention may also be administered prior to a surgical procedure for the purpose of diminishing a size of a tumor (referred to as presurgical adjuvant chemotherapy, or neoadjuvant chemotherapy), or may be administered after a surgical

procedure for the purpose of preventing tumor recurrence (referred to as postsurgical adjuvant chemotherapy, or adjuvant chemotherapy).

In some embodiments, the pharmaceutical composition and the method of treatment of the present invention can also be used as a maintenance therapy. For example, a patient continuously receives the (cancer) therapy for the purpose of preventing recurrence after the first chemotherapy.

As used herein, unless specifically stated otherwise and specifically limited, and so long as it does not contradict technically and in the context, a term written in the singular form is considered to include the plural form, and vice versa.

[Examples]

The present invention is specifically described in view of the examples shown below. However, the present invention is not limited to these. Further, it is by no means to be interpreted in a limited way.

Production Example 1: Production of anti-B7-H3 antibody-drug conjugate

With reference to the production method described in International Publication No. WO 2014/057687 with use of a humanized anti-B7-H3 antibody (an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4), an anti-B7-H3 antibody-drug conjugate in which a drug-linker represented by the following formula:

[Formula 12]

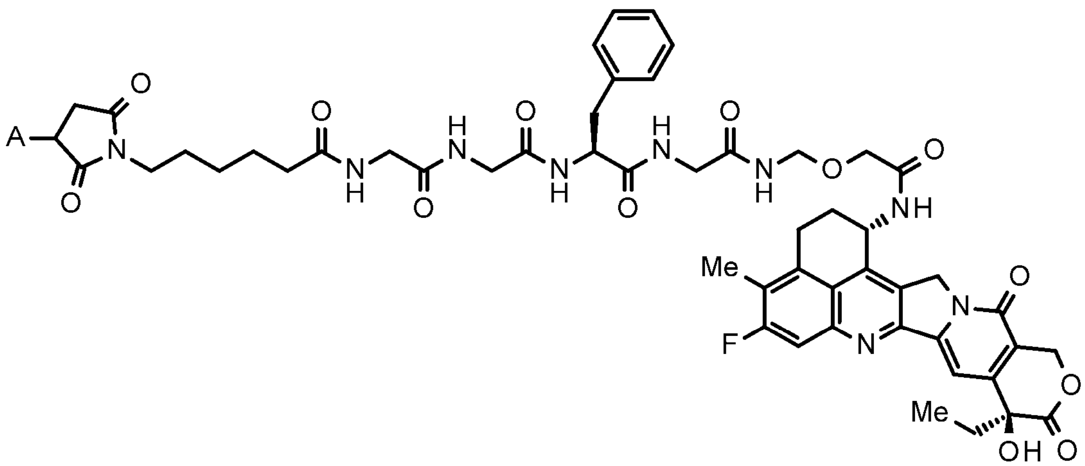

wherein A represents a connecting position to the anti-B7-H3 antibody,
is conjugated to the anti-B7-H3 antibody via a thioether bond (hereinafter, referred to as the "anti-B7-H3 antibody-drug conjugate (1)") was produced. The DAR of the anti-B7-H3 antibody-drug conjugate (1) is 4.0.

Test Example 1: In vitro cell growth inhibition test

The cell growth inhibitory effect was evaluated using human lung cancer cell NCI-H322 and human breast cancer cell line EFM-19.

NCI-H322 and EFM-19 cells were respectively cultured in RPMI 1640 (Life Technologies, Cat. No 11875-119) medium containing 10% (v/v) Fetal Bovine Serum (HyClone, Cat. No SH30396, Lot AE2916535) and 1% (v/v) Antibiotic-Antimycotic (Life Technologies, Cat. No 15240-062) in humidified 5% $CO_2$ environment at 37°C. When each cell line reached 70 to 80% confluence, they were detached using TryPLE (Life Technologies, 12605-010), centrifuged, and then suspended in a medium at a concentration of 1.00 x $10^5$ cells/mL. The prepared cell suspension was seeded in a Corning(R) 96-well flat clear bottom black polystyrene TC-treated microplate, 100 μL each per well, and incubated overnight at 37°C and 5% $CO_2$. On the following day, for the anti-B7-H3 antibody-drug conjugate (1), dilution series of 2000, 600, 200, 60, 20, 6, and 2 nM were prepared using a medium. Additionally, AZD6738 (ATR inhibitor; ATRi, WO2011/154737) and AZD1390 (ATM inhibitor; ATMi, WO2017/046216) were diluted in a medium to be at 600 nM and 200 nM, respectively. For the single agent treated groups of the anti-B7-H3 antibody-drug conjugate (1), each solution of the anti-B7-H3 antibody-drug conjugate (1) dilution series was mixed with the medium in a ratio of 1:1 so that the final

concentrations were 1000, 300, 100, 30, 10, 3, and 1 nM. For the combination agents treated groups, each solution of the anti-B7-H3 antibody-drug conjugate (1) dilution series was mixed with AZD6738 and AZD1390 respectively in a ratio of 1:1 so that each dilution series of the anti-B7-H3 antibody-drug conjugate (1) had the final concentration of 1000, 300, 100, 30, 10, 3, and 1 nM, and the final concentration of AZD6738 or AZD1390 in each dilution series was 300 or 100 nM, respectively. Subsequently, the whole amount of the cell medium incubated overnight was aspirated, and 100 μL of each mixed solution was added to corresponding wells of the 96 well plate containing cells and incubated at 37°C and 5% $CO_2$ for 7 days. Then, the relative viability was quantitatively determined using CellTiter-Glo 2.0 (Promega, G9243) according to the protocol. The results are shown in Figure 14A and Figure 14B. The combination agents treated groups showed a growth inhibitory effect at lower concentration ranges of the anti-B7-H3 antibody-drug conjugate (1) compared to the growth inhibitory curve of the single agent treated groups, thereby demonstrating the enhanced cell growth inhibitory effect by the combination use.

Test Example 2: In vivo antitumor activity of the combination of the anti-B7-H3 antibody-drug conjugate (1) and an ATR inhibitor:

Vehicle 1
10 mM Acetate buffer (pH 5.5), 5% sorbitol
Vehicle 2
10% DMSO, 40% Propylene glycol

Preparation of anti-B7-H3 antibody-drug conjugate (1) dosing solutions

The anti-B7-H3 antibody-drug conjugate (1) is diluted with Vehicle 1 to a concentration of 0.1 mg/mL, 0.3 mg/mL, or 1.0 mg/mL.

Preparation of AZD6738 dosing solutions

The dosing solutions are prepared to a concentration of 1.0 mg/mL, 2.5 mg/mL, or 7.5 mg/mL AZD6738. DMSO (10% of the total volume) is added to AZD6738 and mixed thoroughly using a pellet pestle. Sonication is used for approximately 5 minutes to completely dissolve the compound. Propylene glycol (40% of the total volume) is added to AZD6738 dissolved with DMSO and mixed thoroughly using a magnetic stirrer. Sterilized water (50% of the total volume) is added to this solution and mixed thoroughly using a magnetic stirrer.

Xenograft models:

Model 1

Small cell lung cancer cell line NCI-H446 is purchased from ATCC (American Type Culture Collection). Five to 8-week-old female CAnN.Cg-Foxn1$^{nu}$/CrlCrlj mice (The Jackson Laboratory Japan, Inc.) are subcutaneously injected with 5 × $10^6$ NCI-H446 cells suspended in saline.

Model 2

Small cell lung cancer cell line NCI-H524 is purchased from ATCC. Five to 8-week-old female CAnN.Cg-Foxn1$^{nu}$/CrlCrlj mice (The Jackson Laboratory Japan, Inc.) are subcutaneously injected with 4 × $10^6$ NCI-H524 cells suspended in Matrigel/PBS(-) (1:1).

Model 3

Human prostate cancer epithelial cell line 22Rv1 is purchased from ATCC. Five to 8-week old neutered male NOD.Cg-Prkdc$^{scid}$Il2rg$^{tm1Wjl}$/SzJ mice (The Jackson Laboratory Japan, Inc.) are subcutaneously injected with 5 × $10^6$ 22Rv1 cells suspended in PBS(-) .

When a tumor volume of each model reaches 100 to 300 $mm^3$, the tumor-bearing mice are randomly assigned to the following 16 groups (Day 0).

1. Vehicle group

2. 1 mg/kg Anti-B7-H3 antibody-drug conjugate (1) group
3. 3 mg/kg Anti-B7-H3 antibody-drug conjugate (1) group
4. 10 mg/kg Anti-B7-H3 antibody-drug conjugate (1) group
5. 10 mg/kg AZD6738 group
6. 25 mg/kg AZD6738 group
7. 75 mg/kg AZD6738 group
8. 1 mg/kg Anti-B7-H3 antibody-drug conjugate (1) + 10 mg/kg AZD6738 group
9. 1 mg/kg Anti-B7-H3 antibody-drug conjugate (1) + 25 mg/kg AZD6738 group
10. 1 mg/kg Anti-B7-H3 antibody-drug conjugate (1) + 75 mg/kg AZD6738 group
11. 3 mg/kg Anti-B7-H3 antibody-drug conjugate (1) + 10 mg/kg AZD6738 group
12. 3 mg/kg Anti-B7-H3 antibody-drug conjugate (1) + 25 mg/kg AZD6738 group
13. 3 mg/kg Anti-B7-H3 antibody-drug conjugate (1) + 75 mg/kg AZD6738 group
14. 10 mg/kg Anti-B7-H3 antibody-drug conjugate (1) + 10 mg/kg AZD6738 group
15. 10 mg/kg Anti-B7-H3 antibody-drug conjugate (1) + 25 mg/kg AZD6738 group
16. 10 mg/kg Anti-B7-H3 antibody-drug conjugate (1) + 75 mg/kg AZD6738 group

Vehicle 1 and Vehicle 2 are administered to mice in the vehicle group. The anti-B7-H3 antibody-drug conjugate (1) dosing solutions are administered to mice in the anti-B7-H3 antibody-drug conjugate (1) groups. The AZD6738 dosing solutions are administered to mice in the AZD6738 groups. The anti-B7-H3 antibody-drug conjugate (1) dosing solutions and the AZD6738 dosing solutions are administered to mice in the anti-B7-H3 antibody-drug conjugate (1) + AZD6738 groups. Vehicle 1 is intravenously administered at 10 mL/kg on Day 0 and Day 14. Vehicle 2 is administered orally at 10 mL/kg, twice a day, from Day 0 to Day 14. The anti-B7-H3 antibody-drug conjugate (1) dosing solutions are administered intravenously at 10 mL/kg on Day 0 and Day 14. The AZD6738 dosing solutions are administered orally at 10 mL/kg, twice a day, from Day 0 to Day 14. The antitumor effect will be confirmed by comparing tumor volumes of each group.

Test Example 3: In vivo antitumor activity of the combination of the anti-B7-H3 antibody-drug conjugate (1) and an ATM inhibitor:

Vehicle 1
10 mM Acetate buffer (pH 5.5), 5% sorbitol
Vehicle 3
0.5% HPMC, 0.1% Tween 80

Preparation of anti-B7-H3 antibody-drug conjugate (1) dosing solutions

The anti-B7-H3 antibody-drug conjugate (1) is diluted with Vehicle 1 to a concentration of 0.1 mg/mL, 0.3 mg/mL, or 1.0 mg/mL.

Preparation of AZD1390 dosing solutions

The dosing solutions are prepared to a concentration of 0.3 mg/mL, 1.0 mg/mL, or 3.0 mg/mL AZD1390. First, AZD1390 is dissolved in Vehicle 3 to prepare a solution of 5.0 mg/mL. This solution is stirred overnight and diluted to 0.3 mg/mL, 1.0 mg/mL, or 3.0 mg/mL with Vehicle 3 on the day of administration.

Xenograft models:

Model 1

Small cell lung cancer cell line NCI-H446 is purchased from ATCC. Five to 8-week-old female CAnN.Cg-Foxn1$^{nu}$/CrlCrlj mice (The Jackson Laboratory Japan, Inc.) are subcutaneously injected with $5 \times 10^6$ NCI-H446 cells suspended in saline.

Model 2

Small cell lung cancer cell line NCI-H524 is purchased from ATCC. Five to 8-week-old female CAnN.Cg-Foxn1$^{nu}$/CrlCrlj mice (The Jackson Laboratory Japan, Inc.) are subcutaneously injected with $4 \times 10^6$ NCI-H524 cells

suspended in Matrigel/PBS(-) (1:1).

Model 3

Human prostate cancer epithelial cell line 22Rv1 is purchased from ATCC. Five to 8-week old neutered male NOD.Cg-$Prkdc^{scid}Il2rg^{tm1Wjl}$/SzJ mice (The Jackson Laboratory Japan, Inc.) are subcutaneously injected with $5 \times 10^6$ 22Rv1 Cells suspended in PBS(-).

When a tumor volume of each model reaches 100 to 300 $mm^3$, the tumor-bearing mice are randomly assigned to the following 16 groups (Day 0).

1. Vehicle group
2. 1 mg/kg Anti-B7-H3 antibody-drug conjugate (1) group
3. 3 mg/kg Anti-B7-H3 antibody-drug conjugate (1) group
4. 10 mg/kg Anti-B7-H3 antibody-drug conjugate (1) group
5. 3 mg/kg AZD1390 group
6. 10 mg/kg AZD1390 group
7. 30 mg/kg AZD1390 group
8. 1 mg/kg Anti-B7-H3 antibody-drug conjugate (1) + 3 mg/kg AZD1390 group
9. 1 mg/kg Anti-B7-H3 antibody-drug conjugate (1) + 10 mg/kg AZD1390 group
10. 1 mg/kg Anti-B7-H3 antibody-drug conjugate (1) + 30 mg/kg AZD1390 group
11. 3 mg/kg Anti-B7-H3 antibody-drug conjugate (1) + 3 mg/kg AZD1390 group
12. 3 mg/kg Anti-B7-H3 antibody-drug conjugate (1) + 10 mg/kg AZD1390 group
13. 3 mg/kg Anti-B7-H3 antibody-drug conjugate (1) + 30 mg/kg AZD1390 group
14. 10 mg/kg Anti-B7-H3 antibody-drug conjugate (1) + 3 mg/kg AZD1390 group
15. 10 mg/kg Anti-B7-H3 antibody-drug conjugate (1) + 10 mg/kg AZD1390 group
16. 10 mg/kg Anti-B7-H3 antibody-drug conjugate (1) + 30 mg/kg AZD1390 group

Vehicle 1 and Vehicle 3 are administered to mice in the vehicle group. The anti-B7-H3 antibody-drug conjugate (1) dosing solutions are administered to mice in the anti-B7-H3 antibody-drug conjugate (1) groups. The AZD1390 dosing solutions are administered to mice in the AZD1390 groups. The anti-B7-H3 antibody-drug conjugate (1) dosing solutions and the AZD1390 dosing solutions are administered to mice in the anti-B7-H3 antibody-drug conjugate (1) + AZD1390 groups. Vehicle 1 is administered intravenously at 10 mL/kg on Day 0 and Day 14. Vehicle 3 is administered orally at 10 mL/kg, once a day, from Day 0 to Day 28. The anti-B7-H3 antibody-drug conjugate (1) dosing solutions are administered intravenously at 10 mL/kg on Day 0 and Day 14. The AZD1390 dosing solutions are administered orally at 10 mL/kg, once a day, from Day 0 to Day 28. The antitumor effect will be confirmed by comparing tumor volumes of each group.

Test Example 4: In vivo antitumor activity of the combination of anti-B7-H3 antibody-drug conjugate (1) and an ATR inhibitor

All animal experiments were performed in accordance with the in-house guidelines of the Institutional Animal Care and Use Committee of Daiichi Sankyo Co., Ltd. The human prostate carcinoma epithelial cell line, 22Rv1 was purchased from American Type Culture Collection (ATCC) and cultured in RPMI 1640 Medium (ATCC modification) (Thermo Fisher Scientific) supplemented with 10% heat-inactivated fetal bovine serum (FBS, cytiva) at 37°C in a humidified 5% $CO_2$ atmosphere. On the day of cancer cell inoculation, 22Rv1 cells were trypsinized, washed with D-PBS, and then resuspended in D-PBS at a concentration of $5 \times 10^7$ cells/mL. Castrated male NSG mice (NOD.Cg-$Prkdc^{scid}$ $Il2rg^{tm1Wjl}$/SzJ, 7 weeks of age, The Jackson Laboratory Japan, Inc.) were subcutaneously inoculated with 22Rv1 cells ($5 \times 10^6$ cells/mouse) into the right flank. When the tumor volume reached approximately 200 to 300 $mm^3$, tumor-bearing mice were randomly assigned to the following four groups (day 0, n = 8/group):

Group 1: Vehicle control
Group 2: AZD6738 25 mg/kg
Group 3: Anti-B7-H3 antibody-drug conjugate (1) 3 mg/kg
Group 4: Anti-B7-H3 antibody-drug conjugate (1) 3 mg/kg + AZD6738 25 mg/kg

Anti-B7-H3 antibody-drug conjugate (1) was diluted with Soln. A (10 mmol/L histidine buffer [pH5.9] containing 9% sucrose and 0.02% polysorbate 20) to prepare a 0.3 mg/mL dosing solution. To prepare a 5.0 mg/mL dosing solution, AZD6378 was dissolved in DMSO (10% [v/v] of total volume of the dosing solution), and the solution was sonicated. Then, propylene glycol (40% [v/v] of total volume of the dosing solution) was added to the solution. After mixing with a magnetic

stirrer well, water for injection (50% [v/v] of total volume of the dosing solution) was added to the solution, and then the solution was further mixed well with a magnetic stirrer.

The volume of each dosing solution to be administered was calculated based on the individual body weight measured on the day of dosing. Soln. A as a vehicle control of anti-B7-H3 antibody-drug conjugate (1) was administered intravenously to mice in Group 1 (vehicle control) and Group 2 (AZD6738 25 mg/kg) at 10 mL/kg on days 0 and 15. Soln. B (water for injection containing 10% DMSO and 40% propylene glycol) as a vehicle control of AZD6738 was administered orally to mice in Group 1 (vehicle control) and Group 3 (anti-B7-H3 antibody-drug conjugate (1) 3 mg/kg) at 5 mL/kg twice a day on days 0, 1, 2, 3, 4, 7, 8, 9, 10, and 11. The 0.3 mg/mL dosing solution of anti-B7-H3 antibody-drug conjugate (1) was administered intravenously to mice in Group 3 (anti-B7-H3 antibody-drug conjugate (1) 3 mg/kg) and Group 4 (anti-B7-H3 antibody-drug conjugate (1) 3 mg/kg + AZD6738 25 mg/kg) at 10 mL/kg on days 0 and 15. The 5.0 mg/mL dosing solution of AZD6738 was administered orally to mice in Group 2 (AZD6738 25 mg/kg) and Group 4 (anti-B7-H3 antibody-drug conjugate (1) 3 mg/kg + AZD6738 25 mg/kg) at 5 mL/kg twice a day on days 0, 1, 2, 3, 4, 7, 8, 9, 10, and 11.

Tumor length and tumor width were measured with a digital caliper. Tumor volume and tumor growth inhibition (TGI) were calculated by the following formulas:

$$\text{Tumor volume [mm}^3\text{]} = 1/2 \times (\text{tumor length [mm]}) \times (\text{tumor width [mm]})^2$$

$$\text{TGI [\%]} = (1 - T/C) \times 100$$

C: mean tumor volume of Group 1 (vehicle control)
T: mean tumor volume of a treatment group

Dunnett's test was used to compare tumor volumes in monotherapy groups with those in the vehicle control and to compare tumor volumes in monotherapy groups with those in the combination group. A P value of less than 0.05 was considered to be statistically significant.

In this study, no apparent changes were observed in general conditions or body weight that would be related to the test substances in any groups compared to Group I (vehicle control). One mouse in Group 4 was excluded from the study due to substantial loss of body wight (20.4% loss from baseline [body wight on Day 0]) on day 7. However, 4 out of 8 mice in the vehicle control group also had a loss of body weight of 10% or greater from baseline (maximum: 13.8%), and a similar tendency was also observed in the other groups. Therefore, these observations suggest that loss of body wight in the excluded mouse in Group 4 was due to the vehicles rather than the test substances.

The following calculation of TGI and statistical analyses were performed using data except for the excluded mouse in Group 4.

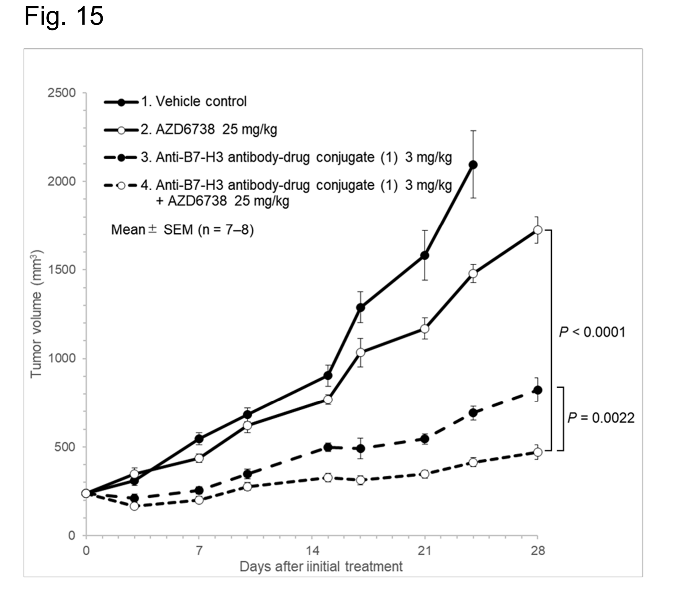

Tumor volumes in the groups are shown in Figure 15. The data represents mean tumor volume ± standard error of the mean (SEM). On day 24, TGIs in Group 2 (AZD6738 25 mg/kg), Group 3 (anti-B7-H3 antibody-drug conjugate (1) 3 mg/kg), and Group 4 (anti-B7-H3 antibody-drug conjugate (1) 3 mg/kg + AZD6738 25 mg/kg) were 29%, 67%, and 80%, respectively. On day 24, monotherapies with AZD6738 (Group 2) and anti-B7-H3 antibody-drug conjugate (1) (Group 3) significantly inhibited tumor growth compared with the vehicle control (Group 1) (*P* = 0.0024 vs. AZD6738, P < 0.0001 vs. anti-B7-H3 antibody-drug conjugate (1)). Furthermore, on day 28, the combination of anti-B7-H3 antibody-drug conjugate (1) and AZD6738 (Group 4) significantly improved antitumor activity compared with AZD6738 alone (Group 2) and anti-B7-H3 antibody-drug conjugate (1) alone (Group 3) (*P* < 0.0001 vs. AZD6738, P = 0.0022 vs. anti-B7-H3 antibody-drug conjugate (1)).

Test Example 5: In vivo antitumor activity of the combination of anti-B7-H3 antibody-drug conjugate (1) and an ATM inhibitor

All animal experiments were performed in accordance with the in-house guidelines of the Institutional Animal Care and Use Committee of Daiichi Sankyo Co., Ltd. The human prostate carcinoma epithelial cell line, 22Rv1 was purchased from American Type Culture Collection (ATCC) and cultured in RPMI 1640 Medium (ATCC modification) (Thermo Fisher Scientific) supplemented with 10% heat-inactivated fetal bovine serum (FBS, cytiva) at 37°C in a humidified 5% $CO_2$ atmosphere. On the day of cancer cell inoculation, 22Rv1 cells were trypsinized, washed with D-PBS, and then resuspended in D-PBS at a concentration of $5 \times 10^7$ cells/mL. Castrated male NSG mice (NOD.Cg-$Prkdc^{scid}$ $Il2rg^{tm1Wjl}$/Szj, 6 weeks of age, The Jackson Laboratory Japan, Inc.) were subcutaneously inoculated with 22Rv1 cells ($5 \times 10^6$ cells/mouse) into the right flank. When the tumor volume reached approximately 200 to 300 $mm^3$, tumor-bearing mice were randomly assigned to the following four groups (day 0, n = 8/group):

Group 1: Vehicle control
Group 2: AZD1390 20 mg/kg
Group 3: Anti-B7-H3 antibody-drug conjugate (1) 3 mg/kg
Group 4: Anti-B7-H3 antibody-drug conjugate (1) 3 mg/kg + AZD1390 20 mg/kg

Anti-B7-H3 antibody-drug conjugate (1) was diluted with Soln. A (10 mmol/L histidine buffer [pH5.9] containing 9% sucrose and 0.02% polysorbate 20) to prepare a 0.3 mg/mL dosing solution. To prepare a 2.0 mg/mL dosing solution, AZD1390 was dissolved in Soln. C (0.5% [w/v] hydroxypropyl methylcellulose [HPMC] and 0.1% [v/v] Tween 80), then the solution was mixed well with a magnetic stirrer.

The volume of each dosing solution to be administered was calculated from the individual body weight measured on the day of dosing. Soln. A as a vehicle control of anti-B7-H3 antibody-drug conjugate (1) was administered intravenously to mice in Group 1 (vehicle control) and Group 2 (AZD1390 20 mg/kg) at 10 mL/kg on days 0 and 14. Soln. C as a vehicle control of AZD1390 was administered orally to mice in Group 1 (vehicle control) and Group 3 (anti-B7-H3 antibody-drug conjugate (1) 3 mg/kg) at 10 mL/kg on days 1, 2, 3, 4, 7, 8, 9, 10, 11, 14, 15, 16, 17, 18, 21, 22, 23, 24, and 25. The 0.3 mg/mL dosing solution of anti-B7-H3 antibody-drug conjugate (1) was administered intravenously to mice in Group 3 (anti-B7-H3 antibody-drug conjugate (1) 3 mg/kg) and Group 4 (anti-B7-H3 antibody-drug conjugate (1) 3 mg/kg + AZD1390 20 mg/kg) at 10 mL/kg on days 0 and 14. The 2.0 mg/mL dosing solution of AZD1390 was administered orally to mice in Group 2 (AZD1390 20 mg/kg) and Group 4 (anti-B7-H3 antibody-drug conjugate (1) 3 mg/kg + AZD1390 20 mg/kg) at 10 mL/kg on days 1, 2, 3, 4, 7, 8, 9, 10, 11, 14, 15, 16, 17, 18, 21, 22, 23, 24, and 25. Tumor length and tumor width were measured with a digital caliper. Tumor volume and tumor growth inhibition (TGI) were calculated by the following formulas:

$$\text{Tumor volume [mm}^3\text{]} = 1/2 \times (\text{tumor length [mm]}) \times (\text{tumor width [mm]})^2$$

$$\text{TGI [\%]} = (1 - T/C) \times 100$$

C: mean tumor volume of Group 1 (vehicle control)
T: mean tumor volume of a treatment group

Dunnett's test was used to compare tumor volumes in monotherapy groups with those in the vehicle control and to compare tumor volumes in monotherapy groups with those in the combination group. A P value of less than 0.05 was considered to be statistically significant.

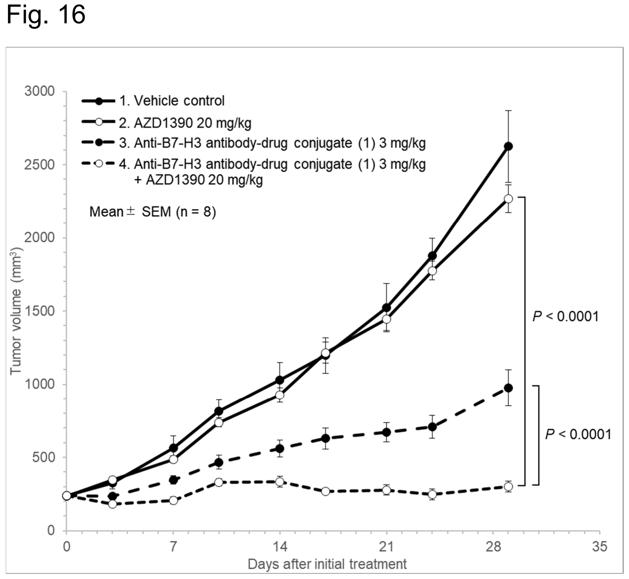

Tumor volumes in the groups is shown in Figure 16. The data represents mean tumor volume ± standard error of the mean (SEM). On day 29, TGIs in Group 2 (AZD1390 20 mg/kg), Group 3 (anti-B7-H3 antibody-drug conjugate (1) 3 mg/kg), and Group 4 (anti-B7-H3 antibody-drug conjugate (1) 3 mg/kg + AZD1390 20 mg/kg) were 14%, 63%, and 89%, respectively. On day 29, monotherapy with anti-B7-H3 antibody-drug conjugate (1) (Group 3) significantly inhibited tumor growth compared with the vehicle control (Group 1) ($P < 0.0001$), but there was no statistically significant difference in antitumor activity between the vehicle control (Group 1) and AZD1390 alone (Group 2) ($P = 0.2466$). On the other hand, on day 29, the combination of anti-B7-H3 antibody-drug conjugate (1) and AZD1390 (Group 4) significantly improved antitumor activity compared with AZD1390 alone (Group 2) and anti-B7-H3 antibody-drug conjugate (1) alone (Group 3) ($P < 0.0001$ vs. AZD1390, P < 0.0001 vs. anti-B7-H3 antibody-drug conjugate (1)).

[Industrial Applicability]

The above experimental results revealed that the antibody-drug conjugate according to the present invention demonstrates excellent antitumor effect when administered in combination with an ATR inhibitor or ATM inhibitor.

[Free Text of Sequence Listing]

SEQ ID NO: 1 - Amino acid sequence of B7-H3 variant 1
SEQ ID NO: 2 - Amino acid sequence of B7-H3 variant 2
SEQ ID NO: 3 - Amino acid sequence of the heavy chain of the anti-B7-H3 antibody (M30-H1 type)
SEQ ID NO: 4 - Amino acid sequence of the light chain of the anti-B7-H3 antibody (M30-L4 type)
SEQ ID NO: 5 - Amino acid sequence of the heavy chain of the anti-B7-H3 antibody (M30-H2 type)

SEQ ID NO: 6 - Amino acid sequence of the heavy chain of the anti-B7-H3 antibody (M30-H3 type)
SEQ ID NO: 7 - Amino acid sequence of the heavy chain of the anti-B7-H3 antibody (M30-H4 type)
SEQ ID NO: 8 - Amino acid sequence of the light chain of the anti-B7-H3 antibody (M30-L1 type)
SEQ ID NO: 9 - Amino acid sequence of the light chain of the anti-B7-H3 antibody (M30-L2 type)
SEQ ID NO: 10 - Amino acid sequence of the light chain of the anti-B7-H3 antibody (M30-L3 type)
SEQ ID NO: 11 - Amino acid sequence of the light chain of the anti-B7-H3 antibody (M30-L5 type)
SEQ ID NO: 12 - Amino acid sequence of the light chain of the anti-B7-H3 antibody (M30-L6 type)
SEQ ID NO: 13 - Amino acid sequence of the light chain of the anti-B7-H3 antibody (M30-L7 type)

## Claims

1. A pharmaceutical composition comprising an anti-B7-H3 antibody-drug conjugate, wherein the anti-B7-H3 antibody-drug conjugate is administered in combination with an ATR inhibitor or an ATM inhibitor; and the anti-B7-H3 antibody-drug conjugate is an antibody-drug conjugate in which a drug-linker represented by the following formula:

[Formula 1]

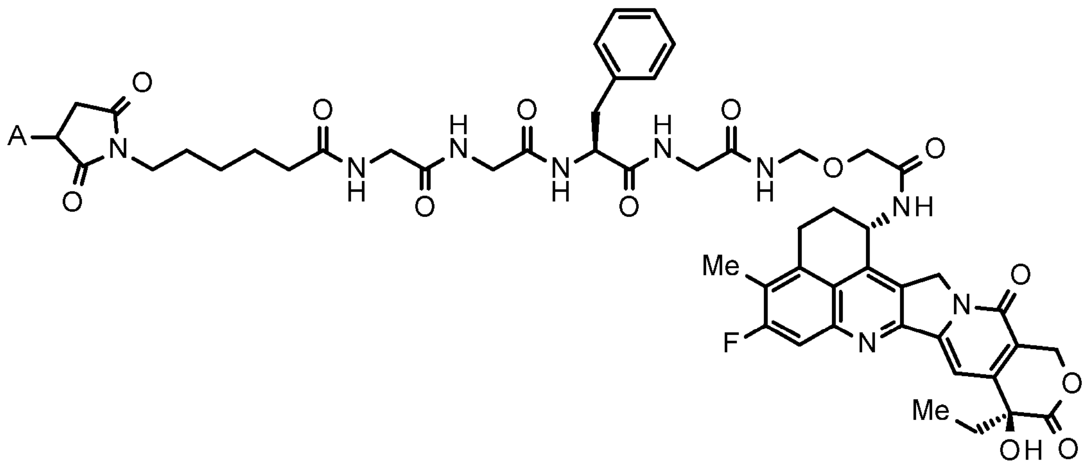

wherein A represents a connecting position to an anti-B7-H3 antibody or functional fragment thereof, and the anti-B7-H3 antibody or functional fragment thereof are conjugated via a thioether bond.

2. The pharmaceutical composition according to claim 1, for treating cancer.

3. The pharmaceutical composition according to claim 1 or 2, wherein the anti-B7-H3 antibody or functional fragment thereof comprises CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, CDRH3 consisting of an amino acid sequence consisting of amino acid residues 118 to 130 of SEQ ID NO: 3, CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

4. The pharmaceutical composition according to any one of claims 1 to 3, wherein the anti-B7-H3 antibody or functional fragment thereof comprises a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 8, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 9, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 10, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 4, a heavy chain variable region consisting of an amino acid sequence consisting of amino acid

residues 20 to 141 of SEQ ID NO: 3 and a light chain variable region consisting of an amino acid sequence consisting of amino acid residues 21 to 128 of SEQ ID NO: 11, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 12, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 13, a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 8, a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 9, a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 10, or a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 4.

**5.** The pharmaceutical composition according to claim 4, wherein the anti-B7-H3 antibody or functional fragment thereof comprises a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 4.

**6.** The pharmaceutical composition according to any one of claims 1 to 4, wherein the anti-B7-H3 antibody comprises a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

**7.** The pharmaceutical composition according to claim 6, wherein the anti-B7-H3 antibody comprises a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

**8.** The pharmaceutical composition according to any one of claims 1 to 7, wherein a lysine residue at the carboxyl terminus of the heavy chain of the anti-B7-H3 antibody is deleted.

**9.** The pharmaceutical composition according to any one of claims 1 to 8, wherein the average number of units of the drug-linker conjugated per antibody molecule in the anti-B7-H3 antibody-drug conjugate is in the range of from 3.5 to 4.5.

**10.** The pharmaceutical composition according to any one of claims 1 to 9, wherein the anti-B7-H3 antibody-drug conjugate is ifinatamab deruxtecan.

**11.** The pharmaceutical composition according to any one of claims 1 to 10, wherein the ATR inhibitor is AZD6738, BAY-1895344 or ETP-46464, or a pharmaceutically acceptable salt thereof.

**12.** The pharmaceutical composition according to any one of claims 1 to 11, wherein the ATR inhibitor is AZD6738 or a pharmaceutically acceptable salt thereof.

**13.** The pharmaceutical composition according to any one of claims 1 to 10, wherein the ATM inhibitor is AZD1390, AZD0156, KU-55933, KU-60019, KU-59403, CP466722 or NVP-BEZ235, or a pharmaceutically acceptable salt thereof.

**14.** The pharmaceutical composition according to any one of claims 1 to 10 or 13, wherein the ATM inhibitor is AZD1390 or a pharmaceutically acceptable salt thereof.

**15.** The pharmaceutical composition according to any one of claims 1 to 14, wherein the anti-B7-H3 antibody-drug conjugate and the ATR inhibitor or ATM inhibitor are contained in separate formulations and are administered at the same time or different times.

**16.** The pharmaceutical composition according to any one of claims 1 to 15, for treating cancer selected from the group consisting of breast cancer, stomach cancer, colorectal cancer, lung cancer, esophageal cancer, head and neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, cervical cancer, squamous cell cancer, peritoneal cancer, liver cancer, hepatocellular cancer, uterine cancer, kidney cancer, vulvar cancer, thyroid cancer, penile cancer, leukemia, malignant lymphoma, plasmacytoma, myeloma, glioblastoma multiforme, osteosarcoma, sarcoma, melanoma, and mesothelioma.

**17.** The pharmaceutical composition according to any one of claims 1 to 16, for treating cancer selected from the group consisting of breast cancer, lung cancer, esophageal cancer, head and neck cancer, prostate cancer, and squamous cell cancer.

**18.** A pharmaceutical composition comprising an anti-B7-H3 antibody-drug conjugate, wherein the anti-B7-H3 antibody-drug conjugate is administered in combination with an ATR inhibitor or an ATM inhibitor, and the anti-B7-H3 antibody-drug conjugate is an antibody-drug conjugate represented by the following formula:

[Formula 2]

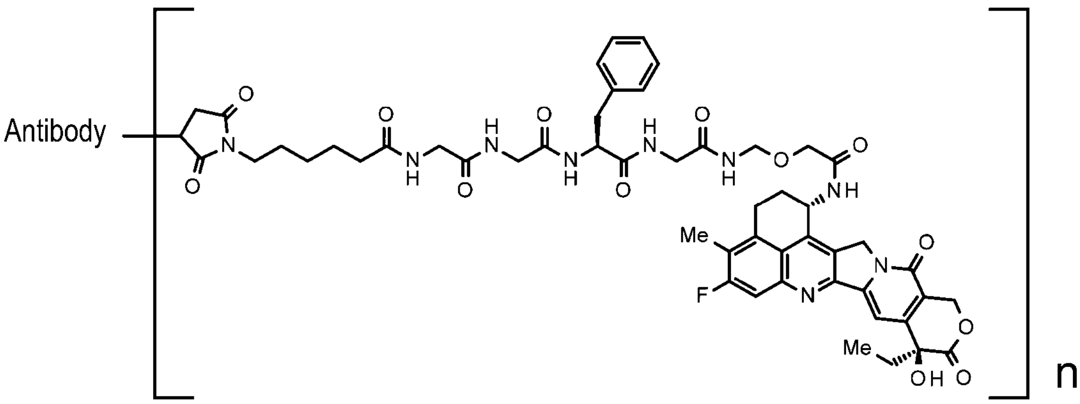

wherein 'Antibody' is an anti-B7-H3 antibody or functional fragment thereof; a drug-linker is conjugated to the antibody or functional fragment thereof via a thioether bond; and n represents the average number of units of the drug-linker conjugated per antibody molecule.

**19.** The pharmaceutical composition according to claim 18, for treating cancer.

**20.** The pharmaceutical composition according to claim 18 or 19, wherein the anti-B7-H3 antibody comprises a heavy chain consisting of an amino acid sequence consisting of amino acid residues 20 to 471 of SEQ ID NO: 3 and a light

chain consisting of an amino acid sequence consisting of amino acid residues 21 to 233 of SEQ ID NO: 4.

**21.** The pharmaceutical composition according to any one of claims 18 to 20, wherein a lysine residue at the carboxyl terminus of the heavy chain of the anti-B7-H3 antibody is deleted.

**22.** The pharmaceutical composition according to any one of claims 18 to 21, wherein n is in the range of from 3.5 to 4.5.

**23.** The pharmaceutical composition according to any one of claims 18 to 22, wherein the anti-B7-H3 antibody-drug conjugate is ifinatamab deruxtecan.

**24.** The pharmaceutical composition according to any one of claims 18 to 23, wherein the ATR inhibitor is AZD6738, BAY-1895344 or ETP-46464, or a pharmaceutically acceptable salt thereof.

**25.** The pharmaceutical composition according to any one of claims 18 to 24, wherein the ATR inhibitor is AZD6738 or a pharmaceutically acceptable salt thereof.

**26.** The pharmaceutical composition according to any one of claims 18 to 23, wherein the ATM inhibitor is AZD1390, AZD0156, KU-55933, KU-60019, KU-59403, CP466722 or NVP-BEZ235, or a pharmaceutically acceptable salt thereof.

**27.** The pharmaceutical composition according to any one of claims 18 to 23 or 26, wherein the ATM inhibitor is AZD1390 or a pharmaceutically acceptable salt thereof.

**28.** The pharmaceutical composition according to any one of claims 18 to 27, wherein the anti-B7-H3 antibody-drug conjugate and the ATR inhibitor or ATM inhibitor are contained in separate formulations and are administered at the same time or different times.

**29.** The pharmaceutical composition according to any one of claims 18 to 28, for treating cancer selected from the group consisting of breast cancer, stomach cancer, colorectal cancer, lung cancer, esophageal cancer, head and neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, cervical cancer, squamous cell cancer, peritoneal cancer, liver cancer, hepatocellular cancer, uterine cancer, kidney cancer, vulvar cancer, thyroid cancer, penile cancer, leukemia, malignant lymphoma, plasmacytoma, myeloma, glioblastoma multiforme, osteosarcoma, sarcoma, melanoma, and mesothelioma.

**30.** The pharmaceutical composition according to any one of claims 18 to 29, for treating cancer selected from the group consisting of breast cancer, lung cancer, esophageal cancer, head and neck cancer, prostate cancer, and squamous cell cancer.

**31.** A method of treatment comprising administering an anti-B7-H3 antibody-drug conjugate in combination with an ATR inhibitor or an ATM inhibitor to a subject in need of treatment, wherein the antibody-drug conjugate is an antibody-drug conjugate in which a drug-linker represented by the following formula:

[Formula 3]

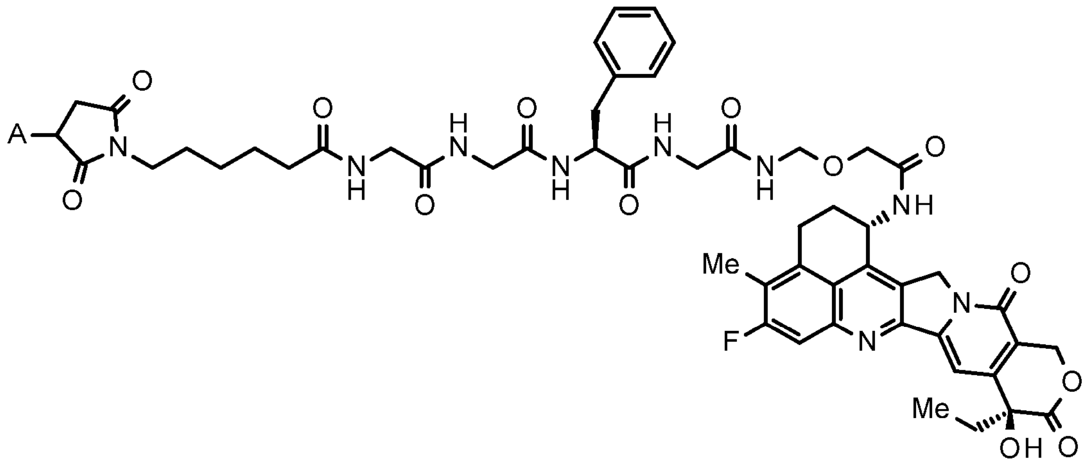

wherein A represents a connecting position to an anti-B7-H3 antibody or functional fragment thereof, and the anti-B7-H3 antibody or functional fragment thereof are conjugated via a thioether bond.

**32.** The method of treatment according to claim 31, wherein the treatment is cancer treatment.

**33.** The method of treatment according to claim 31 or 32, wherein the anti-B7-H3 antibody or functional fragment thereof comprises CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, CDRH3 consisting of an amino acid sequence consisting of amino acid residues 118 to 130 of SEQ ID NO: 3, CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

**34.** The method of treatment according to any one of claims 31 to 33, wherein the anti-B7-H3 antibody or functional fragment thereof comprises a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 8, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 9, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 10, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 4, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 11, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 12, a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 13, a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 8, a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 9, a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 10, or a heavy chain variable region consisting of an amino acid sequence consisting of amino acid residues 20 to 141 of SEQ ID NO: 7 and a light chain variable region consisting of an amino acid sequence consisting of amino acid residues 21 to 128 of SEQ ID NO: 4.

**35.** The method of treatment according to claim 34, wherein the anti-B7-H3 antibody or functional fragment thereof comprises a heavy chain variable region consisting of an amino acid sequence consisting of amino acid residues 20 to 141 of SEQ ID NO: 3 and a light chain variable region consisting of an amino acid sequence consisting of amino acid residues 21 to 128 of SEQ ID NO: 4.

**36.** The method of treatment according to any one of claims 31 to 34, wherein the anti-B7-H3 antibody comprises a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

**37.** The method of treatment according to claim 36, wherein the anti-B7-H3 antibody comprises a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

**38.** The method of treatment according to any one of claims 31 to 37, wherein a lysine residue at the carboxyl terminus of the heavy chain of the anti-B7-H3 antibody is deleted.

**39.** The method of treatment according to any one of claims 31 to 38, wherein the average number of units of the drug-linker conjugated per antibody molecule in the anti-B7-H3 antibody-drug conjugate is in the range of from 3.5 to 4.5.

**40.** The method of treatment according to any one of claims 31 to 39, wherein the anti-B7-H3 antibody-drug conjugate is ifinatamab deruxtecan.

**41.** The method of treatment according to any one of claims 31 to 40, wherein the ATR inhibitor is AZD6738, BAY-1895344 or ETP-46464, or a pharmaceutically acceptable salt thereof.

**42.** The method of treatment according to any one of claims 31 to 41, wherein the ATR inhibitor is AZD6738 or a pharmaceutically acceptable salt thereof.

**43.** The method of treatment according to any one of claims 31 to 40, wherein the ATM inhibitor is AZD1390, AZD0156, KU-55933, KU-60019, KU-59403, CP466722 or NVP-BEZ235, or a pharmaceutically acceptable salt thereof.

**44.** The method of treatment according to any one of claims 31 to 40 or 43, wherein the ATM inhibitor is AZD1390 or a pharmaceutically acceptable salt thereof.

**45.** The method of treatment according to any one of claims 31 to 44, wherein the anti-B7-H3 antibody-drug conjugate and the ATR inhibitor or ATM inhibitor are contained in separate formulations and are administered at the same time or different times.

**46.** The method of treatment according to any one of claims 31 to 45, for treating cancer selected from the group consisting of breast cancer, stomach cancer, colorectal cancer, lung cancer, esophageal cancer, head and neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, cervical cancer, squamous cell cancer, peritoneal cancer, liver cancer, hepatocellular cancer, uterine cancer, kidney cancer, vulvar cancer, thyroid cancer, penile cancer, leukemia, malignant lymphoma, plasmacytoma, myeloma, glioblastoma multiforme, osteosarcoma, sarcoma, melanoma, and mesothelioma.

**47.** The method of treatment according to any one of claims 31 to 46, for treating cancer selected from the group consisting of breast cancer, lung cancer, esophageal cancer, head and neck cancer, prostate cancer, and squamous cell cancer.

**48.** A method of treatment comprising administering an anti-B7-H3 antibody-drug conjugate in combination with an ATR inhibitor or an ATM inhibitor to a subject in need of treatment, wherein the anti-B7-H3 antibody-drug conjugate is an antibody-drug conjugate represented by the following formula:

[Formula 4]

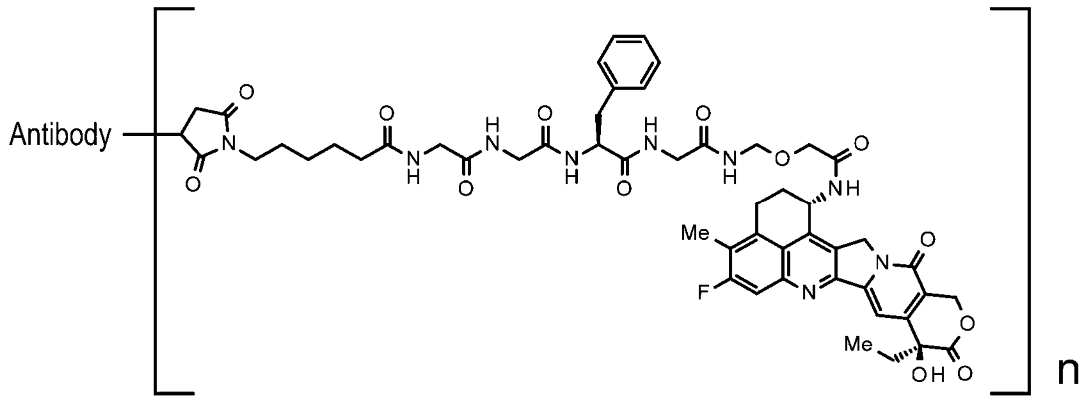

wherein 'Antibody' is an anti-B7-H3 antibody or functional fragment thereof; a drug-linker is conjugated to the antibody or functional fragment thereof via a thioether bond; and n represents the average number of units of the drug-linker conjugated per antibody molecule.

**49.** The method of treatment according to claim 48, wherein the treatment is cancer treatment.

**50.** The method of treatment according to claim 48 or 49, wherein the anti-B7-H3 antibody comprises a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

**51.** The method of treatment according to any one of claims 48 to 50, wherein a lysine residue at the carboxyl terminus of the heavy chain of the anti-B7-H3 antibody is deleted.

**52.** The method of treatment according to any one of claims 48 to 51, wherein n is in the range of from 3.5 to 4.5.

**53.** The method of treatment according to any one of claims 48 to 52, wherein the anti-B7-H3 antibody-drug conjugate is ifinatamab deruxtecan.

**54.** The method of treatment according to any one of claims 48 to 53, wherein the ATR inhibitor is AZD6738, BAY-1895344 or ETP-46464, or a pharmaceutically acceptable salt thereof.

**55.** The method of treatment according to any one of claims 48 to 54, wherein the ATR inhibitor is AZD6738 or a pharmaceutically acceptable salt thereof.

**56.** The method of treatment according to any one of claims 48 to 53, wherein the ATM inhibitor is AZD1390, AZD0156, KU-55933, KU-60019, KU-59403, CP466722 or NVP-BEZ235, or a pharmaceutically acceptable salt thereof.

**57.** The method of treatment according to any one of claims 48 to 53 or 56, wherein the ATM inhibitor is AZD1390 or a pharmaceutically acceptable salt thereof.

**58.** The method of treatment according to any one of claims 48 to 57, wherein the anti-B7-H3 antibody-drug conjugate and the ATR inhibitor or ATM inhibitor are contained in separate formulations and are administered at the same time or different times.

**59.** The method of treatment according to any one of claims 48 to 58, for treating cancer selected from the group consisting of breast cancer, stomach cancer, colorectal cancer, lung cancer, esophageal cancer, head and neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, cervical cancer, squamous cell cancer, peritoneal cancer, liver cancer, hepatocellular cancer, uterine cancer, kidney cancer, vulvar cancer, thyroid cancer, penile cancer, leukemia, malignant lymphoma, plasmacytoma, myeloma, glioblastoma multiforme, osteosarcoma, sarcoma, melanoma, and mesothelioma.

**60.** The method of treatment according to any one of claims 48 to 59, for treating cancer selected from the group consisting of breast cancer, lung cancer, esophageal cancer, head and neck cancer, prostate cancer, and squamous cell cancer.

# Fig. 1

Amino acid sequence of B7-H3 variant 1 (SEQ ID NO: 1)

M L R R R G S P G M G V H V G A A L G A L W F C L T G A L E V Q V P E D P V V A L V G T D
A T L C C S F S P E P G F S L A Q L N L I W Q L T D T K Q L V H S F A E G Q D Q G S A Y A
N R T A L F P D L L A Q G N A S L R L Q R V R V A D E G S F T C F V S I R D F G S A A V S
L Q V A A P Y S K P S M T L E P N K D L R P G D T V T I T C S S Y Q G Y P E A E V F W Q D
G Q G V P L T G N V T T S Q M A N E Q G L F D V H S I L R V V L G A N G T Y S C L V R N P
V L Q Q D A H S S V T I T P Q R S P T G A V E V Q V P E D P V V A L V G T D A T L R C S F
S P E P G F S L A Q L N L I W Q L T D T K Q L V H S F T E G R D Q G S A Y A N R T A L F P
D L L A Q G N A S L R L Q R V R V A D E G S F T C F V S I R D F G S A A V S L Q V A A P Y
S K P S M T L E P N K D L R P G D T V T I T C S S Y R G Y P E A E V F W Q D G Q G V P L T
G N V T T S Q M A N E Q G L F D V H S V L R V V L G A N G T Y S C L V R N P V L Q Q D A H
G S V T I T G Q P M T F P P E A L W V T V G L S V C L I A L L V A L A F V C W R K I K Q S
C E E E N A G A E D Q D G E G E G S K T A L Q P L K I I S D S K E D D G Q E I A

# Fig. 2

Amino acid sequence of B7-H3 variant 2 (SEQ ID NO: 2)

M L R R R G S P G M G V H V G A A L G A L W F C L T G A L E V Q V P E D P V V A L V G T D
A T L C C S F S P E P G F S L A Q L N L I W Q L T D T K Q L V H S F A E G Q D Q G S A Y A
N R T A L F P D L L A Q G N A S L R L Q R V R V A D E G S F T C F V S I R D F G S A A V S
L Q V A A P Y S K P S M T L E P N K D L R P G D T V T I T C S S Y R G Y P E A E V F W Q D
G Q G V P L T G N V T T S Q M A N E Q G L F D V I I S V L R V V L G A N G T Y S C L V R N P
V L Q Q D A H G S V T I T G Q P M T F P P E A L W V T V G L S V C L I A L L V A L A F V C
W R K I K Q S C E E E N A G A E D Q D G E G E G S K T A L Q P L K H S D S K E D D G Q E I
A

## Fig. 3

SEQ ID NO: 3 - Amino acid sequence of the heavy chain of the anti-B7-H3 antibody (M30-H1 type)

M K H L W F F L L L V A A P R W V L S Q V Q L V Q S G A E V K K P G S S V K
V S C K A S G Y T F T N Y V M H W V R Q A P G Q G L E W M G Y I N P Y N D D
V K Y N E K F K G R V T I T A D E S T S T A Y M E L S S L R S E D T A V Y Y
C A R W G Y Y G S P L Y Y F D Y W G Q G T L V T V S S A S T K G P S V F P L
A P S S K S T S G G T A A L G C L V K D Y F P E P V T V S W N S G A L T S G
V H T F P A V L Q S S G L Y S L S S V V T V P S S S L G T Q T Y I C N V N H
K P S N T K V D K R V E P K S C D K T H T C P P C P A P E L L G G P S V F L
F P P K P K D T L M I S R T P E V T C V V V D V S H E D P E V K F N W Y V D
G V E V H N A K T K P R E E Q Y N S T Y R V V S V L T V L H Q D W L N G K E
Y K C K V S N K A L P A P I E K T I S K A K G Q P R E P Q V Y T L P P S R E
E M T K N Q V S L T C L V K G F Y P S D I A V E W E S N G Q P E N N Y K T T
P P V L D S D G S F F L Y S K L T V D K S R W Q Q G N V F S C S V M H E A L
H N H Y T Q K S L S L S P G K

Signal sequence (1-19), Variable region (20-141), Constant region (142-471)

## Fig. 4

SEQ ID NO: 4 - Amino acid sequence of the light chain of the anti-B7-H3 antibody (M30-L4 type)

M V L Q T Q V F I S L L L W I S G A Y G E I V L T Q S P A T L S L S P G E R
A T L S C R A S S R L I Y M H W Y Q Q K P G Q A P R P L I Y A T S N L A S G
I P A R F S G S G S G T D F T L T I S S L E P E D F A V Y Y C Q Q W N S N P
P T F G Q G T K V E I K R T V A A P S V F I F P P S D E Q L K S G T A S V V
C L L N N F Y P R E A K V Q W K V D N A L Q S G N S Q E S V T E Q D S K D S
T Y S L S S T L T L S K A D Y E K H K V Y A C E V T H Q G L S S P V T K S F
N R G E C

Signal sequence (1-20), Variable region (21-128), Constant region (129-233)

# Fig. 5

SEQ ID NO: 5 - Amino acid sequence of the heavy chain of the anti-B7-H3 antibody (M30-H2 type)

MKHLWFFLLLVAAPRWVLSQVQLVQSGAEVKKPGSSVKVSCKASG
YTFTNYVMHWVRQAPGQGLEWIGYINPYNDDVKYNEKFKGRVTIT
ADESTSTAYMELSSLRSEDTAVYYCARWGYYGSPLYYFDYWGQGT
LVTVSSASTKGPSVFPLAPSSKSTSGGTAALGCLVKDYFPEPVTV
SWNSGALTSGVHTFPAVLQSSGLYSLSSVVTVPSSSLGTQTYICN
VNHKPSNTKVDKRVEPKSCDKTHTCPPCPAPELLGGPSVFLFPPK
PKDTLMISRTPEVTCVVVDVSHEDPEVKFNWYVDGVEVHNAKTKP
REEQYNSTYRVVSVLTVLHQDWLNGKEYKCKVSNKALPAPIEKTI
SKAKGQPREPQVYTLPPSREEMTKNQVSLTCLVKGFYPSDIAVEW
ESNGQPENNYKTTPPVLDSDGSFFLYSKLTVDKSRWQQGNVFSCS
VMHEALHNHYTQKSLSLSPGK

Signal sequence (1-19), Variable region (20-141), Constant region (142-471)

# Fig. 6

SEQ ID NO: 6 - Amino acid sequence of the heavy chain of the anti-B7-H3 antibody (M30-H3 type)

MKHLWFFLLLVAAPRWVLSEVQLVQSGAEVKKPGSSVKVSCKASG
YTFTNYVMHWVKQAPGQGLEWIGYINPYNDDVKYNEKFKGKATIT
ADESTSTAYMELSSLRSEDTAVYYCARWGYYGSPLYYFDYWGQGT
LVTVSSASTKGPSVFPLAPSSKSTSGGTAALGCLVKDYFPEPVTV
SWNSGALTSGVHTFPAVLQSSGLYSLSSVVTVPSSSLGTQTYICN
VNHKPSNTKVDKRVEPKSCDKTHTCPPCPAPELLGGPSVFLFPPK
PKDTLMISRTPEVTCVVVDVSHEDPEVKFNWYVDGVEVHNAKTKP
REEQYNSTYRVVSVLTVLHQDWLNGKEYKCKVSNKALPAPIEKTI
SKAKGQPREPQVYTLPPSREEMTKNQVSLTCLVKGFYPSDIAVEW
ESNGQPENNYKTTPPVLDSDGSFFLYSKLTVDKSRWQQGNVFSCS
VMHEALHNHYTQKSLSLSPGK

Signal sequence (1-19), Variable region (20-141), Constant region (142-471)

# Fig. 7

SEQ ID NO: 7 - Amino acid sequence of the heavy chain of the anti-B7-H3 antibody (M30-H4 type)

MKHLWFFLLLVAAPRWVLSEVQLVQSGAEVKKPGSSVKVSCKASG
YTFTNYVMHWVKQAPGQGLEWIGYINPYNDDVKYNEKFKGKATQT
SDKSTSTAYMELSSLRSEDTAVYYCARWGYYGSPLYYFDYWGQGT
LVTVSSASTKGPSVFPLAPSSKSTSGGTAALGCLVKDYFPEPVTV
SWNSGALTSGVHTFPAVLQSSGLYSLSSVVTVPSSSLGTQTYICN
VNHKPSNTKVDKRVEPKSCDKTHTCPPCPAPELLGGPSVFLFPPK
PKDTLMISRTPEVTCVVVDVSHEDPEVKFNWYVDGVEVHNAKTKP
REEQYNSTYRVVSVLTVLHQDWLNGKEYKCKVSNKALPAPIEKTI
SKAKGQPREPQVYTLPPSREEMTKNQVSLTCLVKGFYPSDIAVEW
ESNGQPENNYKTTPPVLDSDGSFFLYSKLTVDKSRWQQGNVFSCS
VMHEALHNHYTQKSLSLSPGK

Signal sequence (1-19), Variable region (20-141), Constant region (142-471)

# Fig. 8

SEQ ID NO: 8 - Amino acid sequence of the light chain of the anti-B7-H3 antibody (M30-L1 type)

MVLQTQVFISLLLWISGAYGEIVLTQSPATLSLSPGERATLSCRA
SSRLIYMHWYQQKPGQAPRLLIYATSNLASGIPARFSGSGSGTDF
TLTISRLEPEDFAVYYCQQWNSNPPTFGQGTKVEIKRTVAAPSVF
IFPPSDEQLKSGTASVVCLLNNFYPREAKVQWKVDNALQSGNSQE
SVTEQDSKDSTYSLSSTLTLSKADYEKHKVYACEVTHQGLSSPVT
KSFNRGEC

Signal sequence (1-20), Variable region (21-128), Constant region (129-233)

# Fig. 9

SEQ ID NO: 9 - Amino acid sequence of the light chain of the anti-B7-H3 antibody (M30-L2 type)

MVLQTQVFISLLLWISGAYGEIVLTQSPATLSLSPGERATLSCRA
SSRLIYMHWYQQKPGQAPRLWIYATSNLASGIPARFSGSGSGTDY
TLTISRLEPEDFAVYYCQQWNSNPPTFGQGTKVEIKRTVAAPSVF
IFPPSDEQLKSGTASVVCLLNNFYPREAKVQWKVDNALQSGNSQE
SVTEQDSKDSTYSLSSTLTLSKADYEKHKVYACEVTHQGLSSPVT
KSFNRGEC

Signal sequence (1-20), Variable region (21-128), Constant region (129-233)

# Fig. 10

SEQ ID NO: 10 - Amino acid sequence of the light chain of the anti-B7-H3 antibody (M30-L3 type)

MVLQTQVFISLLLWISGAYGQIVLSQSPATLSLSPGERATLTCRA
SSRLIYMHWYQQKPGSAPKLWIYATSNLASGIPARFSGSGSGTSY
TLTISRLEPEDFAVYYCQQWNSNPPTFGQGTKVEIKRTVAAPSVF
IFPPSDEQLKSGTASVVCLLNNFYPREAKVQWKVDNALQSGNSQE
SVTEQDSKDSTYSLSSTLTLSKADYEKHKVYACEVTHQGLSSPVT
KSFNRGEC

Signal sequence (1-20), Variable region (21-128), Constant region (129-233)

# Fig. 11

SEQ ID NO: 11 - Amino acid sequence of the light chain of the anti-B7-H3 antibody (M30-L5 type)

MVLQTQVFISLLLWISGAYGQIVLSQSPATLSLSPGERATLTCRA
SSRLIYMHWYQQKPGSAPKPWIYATSNLASGIPARFSGSGSGTSY
TLTISRLEPEDFAVYYCQQWNSNPPTFGQGTKVEIKRTVAAPSVF
IFPPSDEQLKSGTASVVCLLNNFYPREAKVQWKVDNALQSGNSQE
SVTEQDSKDSTYSLSSTLTLSKADYEKHKVYACEVTHQGLSSPVT
KSFNRGEC

Signal sequence (1-20), Variable region (21-128), Constant region (129-233)

# Fig. 12

SEQ ID NO: 12 - Amino acid sequence of the light chain of the anti-B7-H3 antibody (M30-L6 type)

M V L Q T Q V F I S L L L W I S G A Y G E I V L T Q S P A T L S L S P G E R A T L S C R A
S S R L I Y M H W Y Q Q K P G Q A P R P L I Y A T S N L A S G I P A R F S G S G S G T D F
T L T I S R L E P E D F A V Y Y C Q Q W N S N P P T F G Q G T K V E I K R T V A A P S V F
I F P P S D E Q L K S G T A S V V C L L N N F Y P R E A K V Q W K V D N A L Q S G N S Q E
S V T E Q D S K D S T Y S L S S T L T L S K A D Y E K H K V Y A C E V T H Q G L S S P V T
K S F N R G E C

Signal sequence (1-20), Variable region (21-128), Constant region (129-233)

# Fig. 13

SEQ ID NO: 13 - Amino acid sequence of the light chain of the anti-B7-H3 antibody (M30-L7 type)

M V L Q T Q V F I S L L L W I S G A Y G E I V L T Q S P A T L S L S P G E R A T L S C R A
S S R L I Y M H W Y Q Q K P G Q A P R P L I Y A T S N L A S G I P A R F S G S G S G T D Y
T L T I S R L E P E D F A V Y Y C Q Q W N S N P P T F G Q G T K V E I K R T V A A P S V F
I F P P S D E Q L K S G T A S V V C L L N N F Y P R E A K V Q W K V D N A L Q S G N S Q E
S V T E Q D S K D S T Y S L S S T L T L S K A D Y E K H K V Y A C E V T H Q G L S S P V T
K S F N R G E C

Signal sequence (1-20), Variable region (21-128), Constant region (129-233)

Fig. 14A

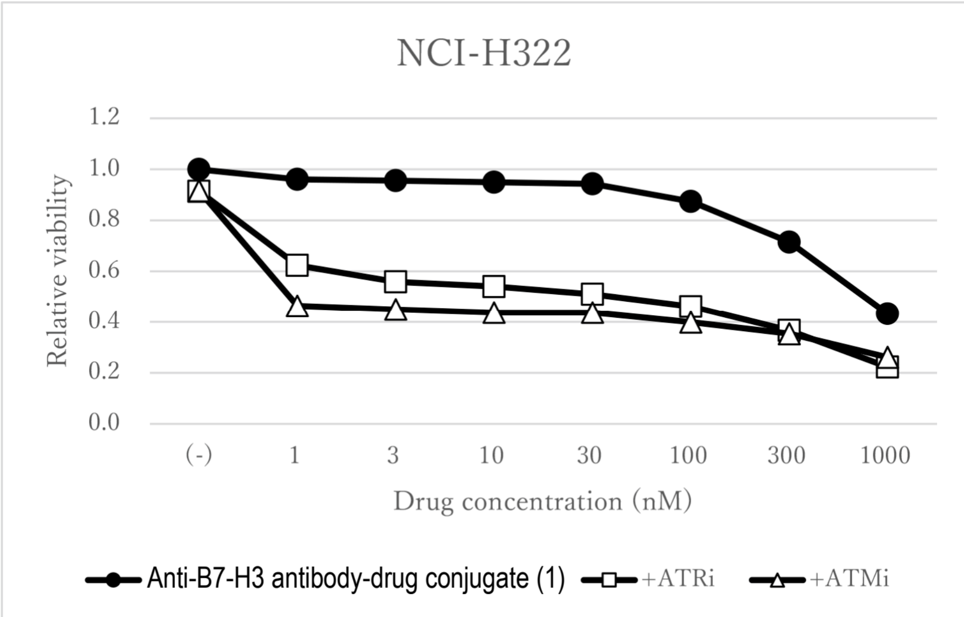


Fig. 14B

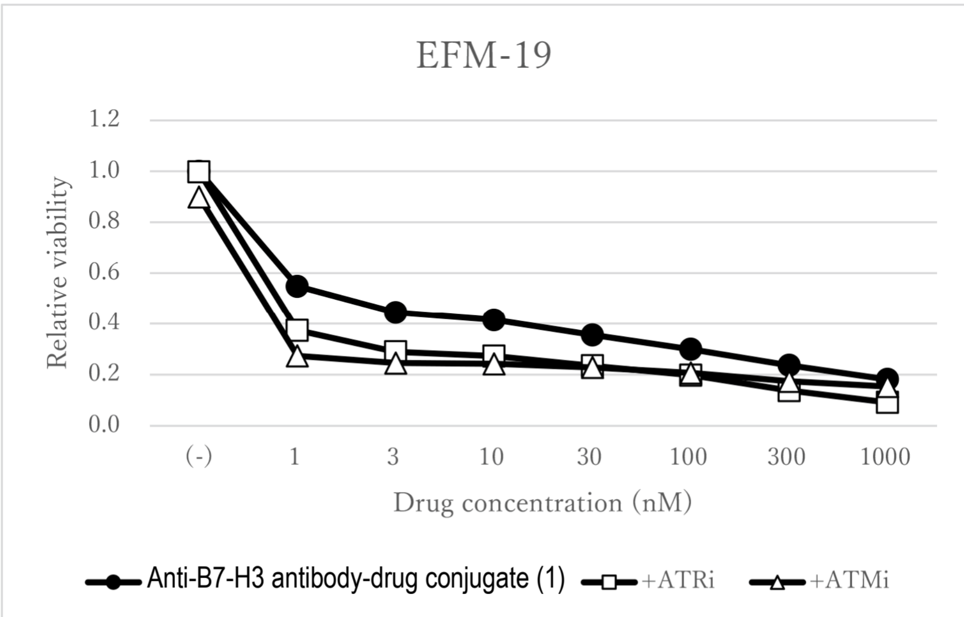

Fig. 15

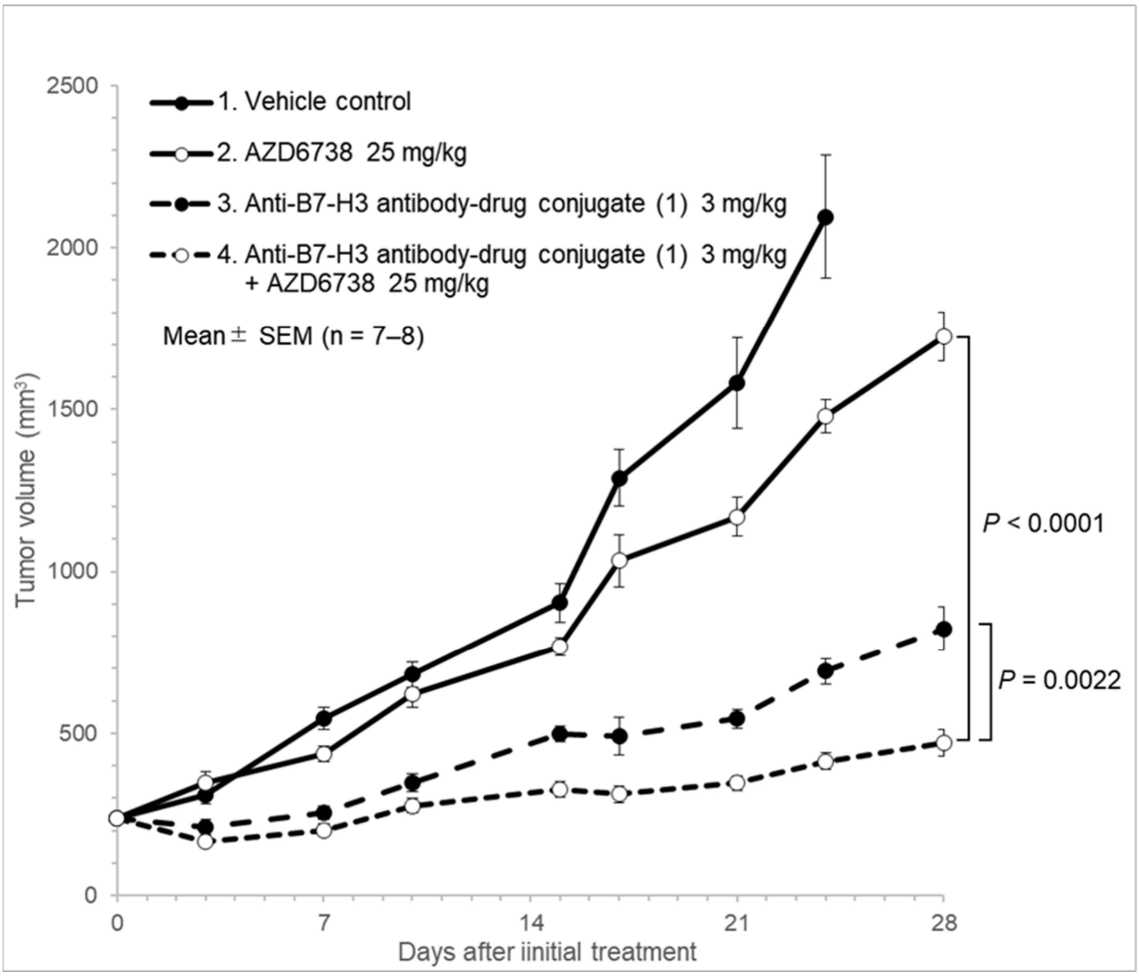

1. Vehicle control
2. AZD6738 25 mg/kg
3. Anti-B7-H3 antibody-drug conjugate (1) 3 mg/kg
4. Anti-B7-H3 antibody-drug conjugate (1) 3 mg/kg + AZD6738 25 mg/kg
Mean ± SEM (n = 7–8)
Tumor volume (mm3)
2500
2000
1500
1000
500
0
0
7
14
21
28
Days after iinitial treatment
P < 0.0001
P = 0.0022

Fig. 16

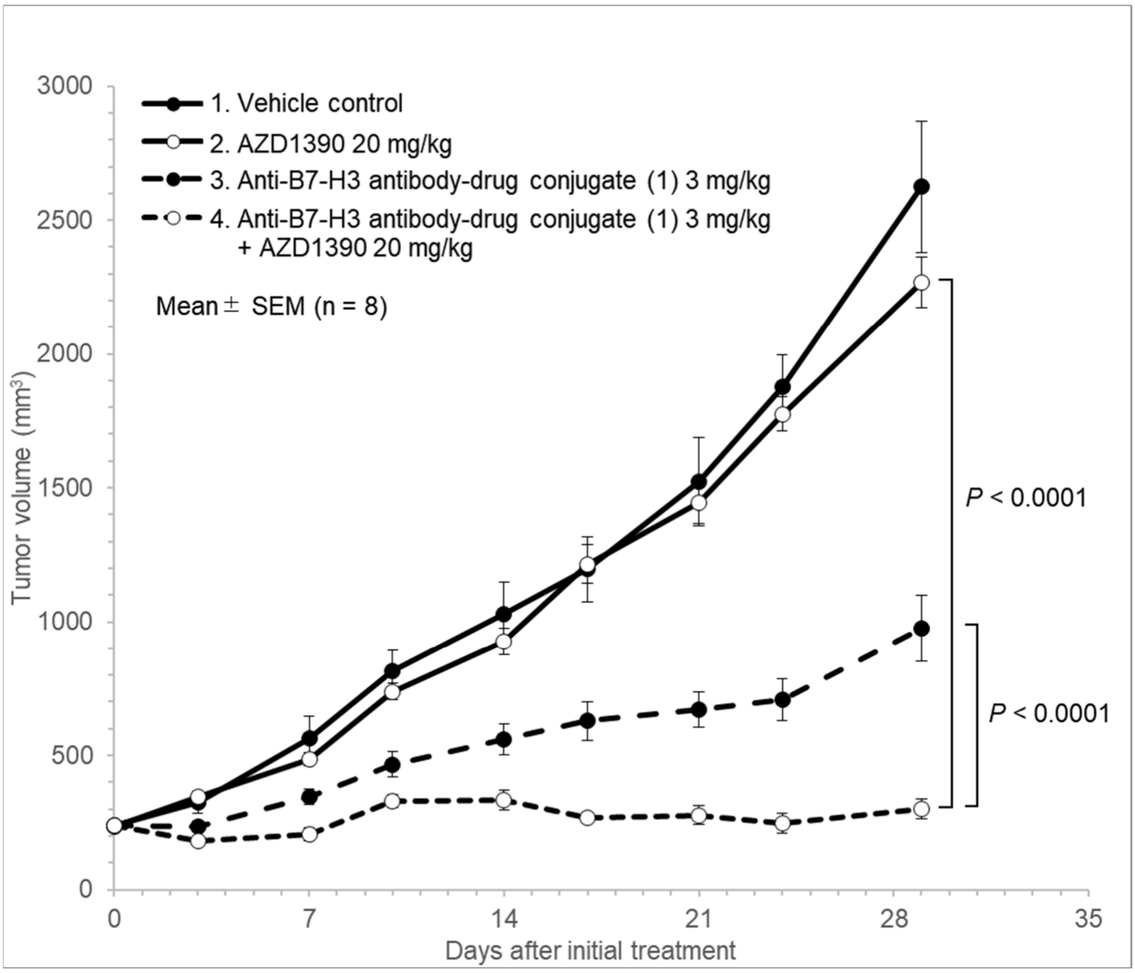
3000
2500
2000
1500
1000
500
0
Tumor volume (mm3)
0
7
14
21
28
35
Days after initial treatment
1. Vehicle control
2. AZD1390 20 mg/kg
3. Anti-B7-H3 antibody-drug conjugate (1) 3 mg/kg
4. Anti-B7-H3 antibody-drug conjugate (1) 3 mg/kg + AZD1390 20 mg/kg
Mean ± SEM (n = 8)
P < 0.0001
P < 0.0001

## INTERNATIONAL SEARCH REPORT

International application No. **PCT/JP2024/014351**

### A. CLASSIFICATION OF SUBJECT MATTER

***A61K 47/68***(2017.01)i; ***A61K 31/517***(2006.01)i; ***A61K 31/4745***(2006.01)i; ***A61K 31/5377***(2006.01)i; ***A61K 31/5383***(2006.01)i; ***A61K 39/395***(2006.01)i; ***A61K 45/00***(2006.01)i; ***A61K 47/65***(2017.01)i; ***A61P 1/00***(2006.01)i; ***A61P 1/16***(2006.01)i; ***A61P 1/18***(2006.01)i; ***A61P 11/00***(2006.01)i; ***A61P 13/00***(2006.01)i; ***A61P 13/08***(2006.01)i; ***A61P 13/10***(2006.01)i; ***A61P 13/12***(2006.01)i; ***A61P 15/00***(2006.01)i; ***A61P 17/00***(2006.01)i; ***A61P 19/00***(2006.01)i; ***A61P 21/00***(2006.01)i; ***A61P 35/00***(2006.01)i; ***A61P 35/02***(2006.01)i; ***A61P 43/00***(2006.01)i; ***C07K 16/28***(2006.01)i; ***C12N 15/13***(2006.01)i

FI: A61K47/68; A61P1/00; A61P1/16; A61P1/18; A61P11/00; A61P13/00; A61P13/08; A61P13/10; A61P13/12; A61P15/00; A61P17/00; A61P19/00; A61P35/00; A61P35/02; A61P43/00 105; A61P21/00; A61K31/4745; A61P43/00 121; A61K45/00; A61K39/395 T; A61K31/5377; A61K31/5383; A61K31/517; C07K16/28; C12N15/13; A61K47/65 ZNA

According to International Patent Classification (IPC) or to both national classification and IPC

### B. FIELDS SEARCHED

Minimum documentation searched (classification system followed by classification symbols)

A61K47/68; A61K31/517; A61K31/4745; A61K31/5377; A61K31/5383; A61K39/395; A61K45/00; A61K47/65; A61P1/00; A61P1/16; A61P1/18; A61P11/00; A61P13/00; A61P13/08; A61P13/10; A61P13/12; A61P15/00; A61P17/00; A61P19/00; A61P21/00; A61P35/00; A61P35/02; A61P43/00; C07K16/28; C12N15/13

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2022/102695 A1 (DAIICHI SANKYO CO., LTD.) 19 May 2022 (2022-05-19) claims, examples, paragraph [0115] | 1-60 |
| Y | WO 2021/260579 A1 (ASTRAZENECA UK LIMITED) 30 December 2021 (2021-12-30) claims, examples | 1-60 |
| Y | WO 2021/260580 A1 (ASTRAZENECA UK LIMITED) 30 December 2021 (2021-12-30) claims, examples | 1-60 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 June 2024** | **02 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

**INTERNATIONAL SEARCH REPORT**

International application No.
**PCT/JP2024/014351**

**C. DOCUMENTS CONSIDERED TO BE RELEVANT**

| Category* | Citation of document, with indication, where appropriate, of the relevant passages | Relevant to claim No. |
|---|---|---|
| Y | LIU, S. et al. The Role of CD276 in Cancers. Frontiers in Oncology. 2021, vol. 11, Article 654684, pp. 1-11<br>table 1 | 1-60 |

Form PCT/ISA/210 (second sheet) (July 2022)

**INTERNATIONAL SEARCH REPORT**

International application No.
**PCT/JP2024/014351**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/014351**

| Patent document cited in search report | Publication date (day/month/year) | Patent family member(s) | Publication date (day/month/year) |
|---|---|---|---|
| WO 2022/102695 A1 | 19 May 2022 | US 2023/0398230 A1<br>claims, examples, paragraph [0135]<br>EP 4245322 A1<br>CN 116615250 A<br>KR 10-2023-0107239 A<br>TW 202233249 A | |
| WO 2021/260579 A1 | 30 December 2021 | JP 2023-542066 A<br>US 2023/0330243 A1<br>EP 4171650 A1<br>CN 116635082 A<br>KR 10-2023-0043109 A | |
| WO 2021/260580 A1 | 30 December 2021 | JP 2023-542066 A<br>US 2023/0330243 A1<br>EP 4171650 A1<br>CN 116635082 A<br>KR 10-2023-0043109 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014057687 A
- WO 2023061457 A
- US 11685742 B2
- US 20210347894 A
- US 20220233708 A
- WO 2024022372 A
- WO 2022117040 A
- WO 2024037503 A
- WO 2023241663 A
- WO 9007861 A
- US 5821337 A
- WO 9954342 A
- WO 0061739 A
- WO 0231140 A
- WO 2007133855 A
- WO 2013120066 A
- WO 2015098099 A
- WO 2015115091 A
- WO 2015155998 A
- WO 2019044947 A
- WO 2017002776 A
- WO 2011154737 A
- WO 2017046216 A
- WO 2015170081 A
- WO 2003070726 A1
- WO 2005016919 A
- WO 2006122806 A

### Non-patent literature cited in the description

- *Cancer Treat. Rev.*, 2017, vol. 60, 139-151
- **OGITANI Y. et al.** *Clinical Cancer Research*, 29 March 2016, vol. 22 (20), 5097-5108
- **OGITANI Y. et al.** *Cancer Science*, 2016, vol. 107, 1039-1046
- *Cell Death and Differentiation*, 2008, vol. 15, 751-761
- *Molecular Biology of the Cell*, December 2004, vol. 15, 5268-5282
- *Bio Techniques*, January 2000, vol. 28, 162-165
- **KOHLER** ; **MILSTEIN**. *Nature*, 1975, vol. 256, 495-497
- Monoclonal Antibodies. Plenum Press, 1980, 365-367
- *Proc. Natl. Acad. Sci. USA*, 1984, vol. 81, 6851-6855
- *Nature*, 1986, vol. 321, 522-525
- **TOMIZUKA, K. et al.** *Nature Genetics*, 1997, vol. 16, 133-143
- **KUROIWA, Y.** *Nucl. Acids Res.*, 1998, vol. 26, 3447-3448
- **YOSHIDA, H.** Animal Cell Technology: Basic and Applied Aspects. Kluwer Academic Publishers, 1999, vol. 10, 69-73
- **TOMIZUKA, K.** *Proc. Natl. Acad. Sci. USA*, 2000, vol. 97, 722-727
- **WORMSTONE, I. M.** *Investigative Ophthalmology & Visual Science*, 2002, vol. 43 (7), 2301-2308
- **CARMEN, S.** *Briefings in Functional Genomics and Proteomics*, 2002, vol. 1 (2), 189-203
- **SIRIWARDENA, D.** *Ophthalmology*, 2002, vol. 109 (3), 427-431
- *Journal of Chromatography A*, 1995, vol. 705, 129-134
- *Analytical Biochemistry*, 2007, vol. 360, 75-83
- **ALTSCHUL, STEPHEN F.** ; **THOMAS L. MADDEN** ; **ALEJANDRO A. SCHAFFER** ; **JINGHUI ZHANG** ; **ZHENG ZHANG** ; **WEBB MILLER** ; **DAVID J. LIPMAN**. Gapped BLAST and PSI-BLAST: a new generation of protein database search programs. *Nucleic Acids Res.*, 1997, vol. 25, 3389-3402
- **HERMANSON, G. T**. Bioconjugate Techniques. Academic Press, 1996, 56-136, 456-493
- *CHEMICAL ABSTRACTS*, 1876467-74-1
- *CHEMICAL ABSTRACTS*, 1345675-02-6
- *CHEMICAL ABSTRACTS*, 587871-26-9
- *CHEMICAL ABSTRACTS*, 925701-49-1
- *CHEMICAL ABSTRACTS*, 1080622-86-1
- *CHEMICAL ABSTRACTS*, 915019-65-7